# EUROPEAN PATENT SPECIFICATION

(11) **EP 0 347 802 B1**
(45) Date of publication and mention of the grant of the patent: **15.06.1994**
(21) Application number: 89111108.0
(22) Date of filing: 19.06.1989
(51) Int. Cl.: C07K 7/02, C07K 1/00, A61K 37/02

(54) **Neurokinin A antagonists**
Neurokinin-A-Antagonisten
Antagonistes de la neurokinine A

(30) Priority: 20.06.1988 US 208926
(43) Date of publication of application: 27.12.1989
(73) Proprietor: MERRELL DOW PHARMACEUTICALS INC., Cincinnati Ohio 45215-6300 (US)
(72) Inventor: Buck, Stephen H., Cincinnati Ohio 45215 (US); Krstenansky, John L., Cincinnati Ohio 45208 (US); McCarthy, James R., West Chester Ohio 45069 (US); Harbeson, Scott L., Cincinnati Ohio 45202 (US)
(74) Representative: VOSSIUS & PARTNER

(56) References cited:
- PROC. EUR. PEPT. SYMP., 18TH, 1984, pages 369-372, Ulf Ranarsson, Stockholm, SE; P. SANDBERG et al.: "Synthesis of cyclic analogs based on substance P"
- EUR. J. PHARMACOL, vol. 118, no. 1-2, 1985, pages 25-36, Elsevier Science Publishers B.V.; J. MIZRAHI et al.: "Tachycinin receptors in smooth muscels: a study with agonists (subst. P, neurokinin A) and antagonists"

## Description

This invention relates to novel peptide derivatives which are antagonists of neurokinin A.

Substance P and related tachykinins, neurokinin A and neurokinin B, are a group of naturally occurring peptides shown to have wide distribution within body tissue and a wide variety of biological effects. While agonists and antagonists of substance P and neurokinin B are known and while agonists of neurokinin A are known as well, antagonists of neurokinin A have not yet been reported. Applicants have now discovered a class of neurokinin A antogonists. Such compounds are not only interesting from a biochemical viewpoint, but such compounds also have valuable pharmacological and medical utilities.
Peptide derivatives of the following structure 1 are antagonists of neurokinin A:

X-A₁-A₂-A₃-A₄-A₅-A₆-Y 1

wherein
- X: is hydrogen, an alkyl group of from 1 to 6 carbon atoms, or an acyl group of from 2 to 10 carbon atoms;
- A₁: is a bond or is a group consisting of from 1 to 4 amino acids;
- A₂: is a bond or is Asp or Glu;
- A₃: is any amino acid;
- A₄: is Phe or N-Me-Phe;
- A₅: is Ile, Val, Leu, Phe, Ala, Tyr, Nle, Met, or N-Me-Val;
- A₆: is Gly or Sar; and
- Y: is a group of the formula 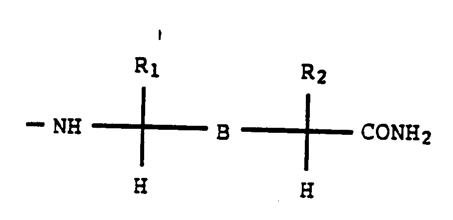 wherein
- B: is a group of one of the formulae 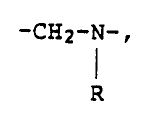 -CH₂-S-,
-CH₂-O-,
-CH=CH-, 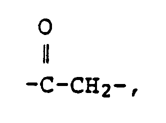 -CH(OH)CH₂-, and 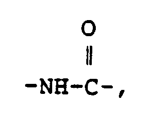 and wherein
- R: is a hydrogen atom or an alkyl group of from 1 to 4 carbon atoms or is a phenylalkylene group wherein the alkylene moiety is straight or branched and has from 1 to 6 carbon atoms and wherein the phenyl moiety is unsubstituted or is mono substituted with a C₁₋₄ alkyl, C₁₋₄ alkoxy, hydroxy, or halogen group;
- R₁ and R₂: are each independently selected from isopropyl, isobutyl, sec-butyl, n-butyl, and 2-(methylthio)ethyl groups
or a pharmaceutically acceptable salt thereof. These novel peptide derivatives are antagonists of neurokinin A and are thus useful antiasthma, antiinflammatory, and antiarthritic agents.

The following common abbreviations of the amino acids and amino and carboxy terminal groups are used throughout this specification:
Gly (or G) - glycine
Ala (or A) - alanine
Val (or V) - valine
Leu (or L) - leucine
Ile (or I) - isoleucine
Fum - fumaryl
Orn - ornithine
Pro (or P) - proline
Phe (or F) - phenylalanine
Trp (or W) - tryptophan
Met (or M) - methionine
Ser (or S) - serine
Thr (or T) - threonine
Cys (or C) - cysteine
Tyr (or Y) - tyrosine
Asn (or N) - asparagine
Gln (or Q) - glutamine
Asp (or D) - aspartic acid
Glu (or E) - glutamic acid
Lys (or K) - lysine
Arg (or R) - arginine
His (or H) - histidine
Nle - norleucine
Hyp - hydroxyproline
Glt - glutaryl
Mal - maleyl
Npa -β-(2-naphthyl)alanine
3,4-dehydroPro - 3,4-dehydroproline
Pgl - phenylglycine
NMePgl - N-methyl-phenylglycine
Sar - sarcosine (N-methylglycine)
pSubPhe - para substituted phenylalanine
SubPhe - ortho, meta, or para, mono- or di-
substituted phenylalanine
DAla (or a) - D-alanine
Ac - acetyl
Suc - succinyl
pClPhe - para-chloro-phenylalanine
pNO₂Phe - para-nitro-phenylalanine
NMeVal - N-methyl-valine
An alkyl group and the alkyl portion of an alkoxy group are taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. The alkylene moiety of the phenyl-alkylene groups of this invention can contain from 1 to 4 carbon atoms and may be straight or branched, for example, methylene, ethylene, propylene, butylene, isopropylidene, and sec-butylidene. The phenyl moiety of the phenylalkylene groups of this invention can be unsubstituted or can be mono substituted at the ortho, meta, or preferably the para positions. Unsubstituted phenyl or para hydroxyphenyl are preferred. An acyl group of from 2 to 10 carbon atoms is taken to include straight, branched, cyclic, saturated and unsaturated acyl groups having 1 or 2 carbonyl moieties per group, for example, acetyl, benzoyl, succinyl, maleyl, and glutaryl. A halogen group is a fluoro, chloro, bromo, or iodo group. In the X groups of this invention, the hydrogen, alkyl, or acyl moiety is attached to the alpha amino group of the amino terminal amino acid. Those peptides wherein the amino group of the amino terminal amino acid is substituted with two alkyl or acyl groups are also considered to be within the scope of the peptides of this invention.

It should be apparent that the peptide derivatives of this invention involve peptides wherein the normal peptide amide bond of the two carbon terminal amino acids of naturally occurring neurokinin A have been modified and these two modified amino acids are chemically depicted herein as the group Y. Utilizing conventional nomenclature employed by peptide chemists, the Y group which is comprised of two Leu residues (i.e., wherein R₁ and R₂ are each a sec-butyl group) having as their amide linkage modified by reducing the carbonyl group to a methylene group, can be designated as LeuΨ[CH₂NH]Leu. This designation indicates that the amide carbonyl group of the penultimate Leu is reduced to a methylene group. Other nomenclature designations used to describe the peptide derivatives of this invention are Ψ[CH₂S], Ψ[CH₂O], Ψ[CH=CH], Ψ[C(O)CH₂], Ψ[CH(OH)CH₂], and Ψ[NHC(O)].

The term "a bond" when used in relation to the definition of A₁ is intended to mean that the X group is directly bonded to the A₂ group or in the instances where A₂ is also a bond, X is then directly bonded to the A₃ group. Likewise the term "a bond" when used in relation to the definition of A₂ is intended to mean that A₁ is directly bonded to the A₃ group or in the instances where A₁ is also a bond, X is then directly bonded to the A₃ group.

The term "any amino acid" as used herein includes the naturally occurring amino acids as well as other "non-protein" α-amino acids commonly utilized by those in the peptide chemistry arts when preparing synthetic analogs of naturally occurring peptides. The naturally occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines substituted at the ortho, meta, or paraposition of the phenyl moiety with one or two of the following, a (C₁-C₄) alkyl, (C₁-C₄) alkoxy, halogen, or nitro groups or substituted with a methylenedioxy group, β-2- and 3-thienylalanine, β-2- and 3-furanylalanine, β-2-, 3-, and 4-pyridylalanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodo-tyrosine and the D-isomers of the naturally occurring amino acids.

The natural amino acids with the exception of glycine, contain a chiral carbon atom. Unless otherwise specifically indicated, the optically active amino acids, referred to herein, are of the L-configuration. As is customary, the structure of peptides written out herein is such that the amino terminal end is on the left side of the chain and the carboxy terminal end is on the right side of the chain. Consistent with this and with customary usage, the B groups are drawn such that the open valence on the left side is attached to the carbon atom of the Y group bearing an "H", the "R₁" group, and an "NH" group and the open valence on the right hand side of the B group is attached to the carbon atom of the Y group bearing an "H", the "R₂" group, and an "CONH₂".

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N′-dibenzylethylenediamine, dihydrodiethylamine, N-(lower alkyl)piperidine, and any other suitable amine.

As with any generic group of chemical compounds, certain groups are preferred. Applicants prefer those peptide derivatives of formula 1 wherein X is a hydrogen and where A₁ is a bond, applicants prefer those peptide derivatives of formula 1 wherein X is Glt, Mal, Fum, and especially Suc. Applicants also prefer those peptide derivatives of formula 1 wherein A₁ is His-Lys-Thr, Lys-Thr, Thr, Asp-Val-Pro-Lys-Ser, Val-Pro-Lys-Ser, Pro-Lys-Ser, Lys-Ser, Ser, pGlu-Pro-Ser-Lys, Pro-Ser-Lys, Ser-Lys, or Lys. Applicants especially prefer those peptide derivatives of formula 1 wherein A₁ is His-Lys-Thr, Lys-Thr, or Thr. Applicants prefer those peptide derivatives of formula 1 wherein A₂ is Asp. Applicants also prefer those peptide derivatives of formula 1 wherein A₃ is Gly, Gln, Asn, Sar and especially Ala or Ser. Applicants further prefer those peptide derivatives of formula 1 wherein A₄ is Phe as well as those peptide derivatives of formula 1 wherein A₅ is Val and wherein A₆ is Gly. Applicants prefer those peptide derivatives of formula 1 wherein B is -CH₂NH- as well as those wherein R₁ is isobutyl, that is 2-methylpropyl, and wherein R₂ is 2-methylthioethyl, isobutyl, or n-butyl. Applicants especially prefer those compounds wherein R₁ and R₂ are each an isobutyl. The most preferred peptide derivatives of formula 1 are H-Asp-Ser-Phe-Val-Gly-LeuΨ[CH₂NR]-Leu-NH₂ wherein R is H or a methyl group.

The proteins of this invention can be prepared by a variety of procedures readily known to those skilled in the art. Such procedures include the solid phase sequential procedure which can be performed using established automated methods such as by use of an automated peptide sythesizer. To prepare the peptide derivatives of this invention, a modified dipeptide corresponding to the carbon terminal dipeptide having the modified peptide linkage or its precursor is bound to a resin support. Procedures to be employed to prepare each of the modified peptide linkages are well known in the art and can be readily performed by skilled peptide chemists. The procedure to prepare those peptide derivatives of formula 1 wherein B is a -NHCO- group, that is the Ψ[NHCO] compounds, is known from Chorev and Goodman, Int. J. Pept. Protein Res., 21(3), 258-68 (1983). The procedure to prepare those peptide derivatives of formula 1 wherein B is a -COCH₂- or -CH(OH)CH₂- group, that is the Ψ[COCH₂] and Ψ[CH(OH)CH₂] compounds, respectively, is known from Holladay and Rich, Tetrahedron Letters, 24(41), 4401-04, (1983). The procedure to prepare those peptide derivatives of formula 1 wherein B is a -CH₂NH- group, that is the Ψ[CH₂NH] compounds, is known from Sasaki and Coy, Peptides, Vol. 8, pp. 119-121, 1987 and is more fully described below. The procedure to prepare those peptide derivatives of formula 1 wherein B is a -CH₂S-group, that is the Ψ[CH₂S] compounds, is known from Spatola and Darlak, Tetrahedron Letters, 44(3), 821-33 (1988). The procedure to prepare those peptide derivatives of formula 1 wherein B is a -CH₂O- group, that is the Ψ[CH₂O] compounds, is known from TenBrink, J. Org. Chem., 1987, 52, 418-22.

Specifically, the compounds of this invention wherein B is a -CH₂N(R)- group are prepared by reducing the N-methoxy-N-methylamide of formula 2 to produce the aldehyde of formula 3. The reduction can be performed in any way generally known and readily performed by those skilled in the art such as by use of lithium aluminum hydride (LAH). This reduction can be conveniently carried out by adding about one molar equivalent of LAH to a cooled, typically about 0°C, solution of a formula 2 compound in a nonreactive solvent such as an ethereal solvent such as tetrahydrofuran (THF) or diethylether. After the reaction is substantially complete, typically after about 30 minutes, the reaction mixture is quenched by the addition of, for example, 10% potassium or sodium hydrogen sulfate and then water. The product can then be isolated by, for example, extraction of the aqueous mixture with a solvent such as diethylether, washing the ether phase with cold, dilute aqueous hydrochloric acid, drying and solvent removal. The crude product can be purified by, for example, column chromatography such as a silica gel column eluting with 55% ethyl acetate/hexane. 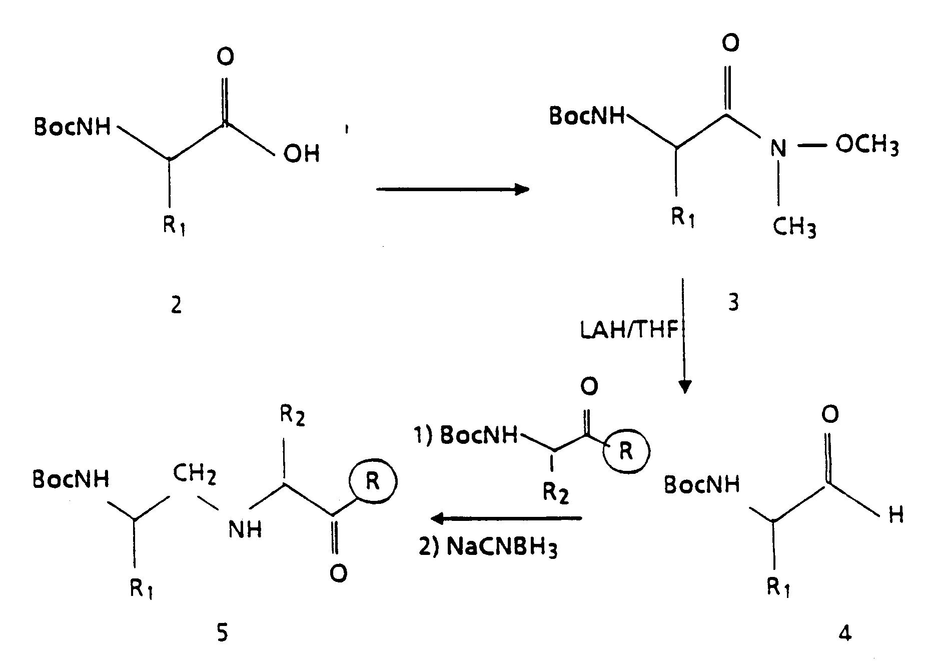
The formula 3 aldehyde is then reacted with a resin-bound amino acid of formula 6 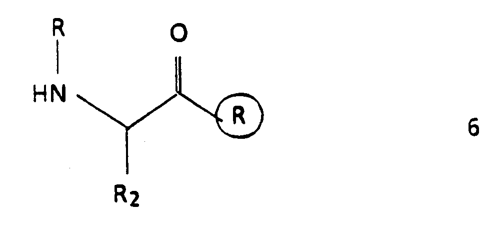
wherein R and R₂ are as defined for formula 1 and wherein Ⓡ represents the resin. The initially formed Schiff base adduct is reduced in situ using, for example, sodium cyanoborohydride, to give a resin bound modified dipeptide of formula 7 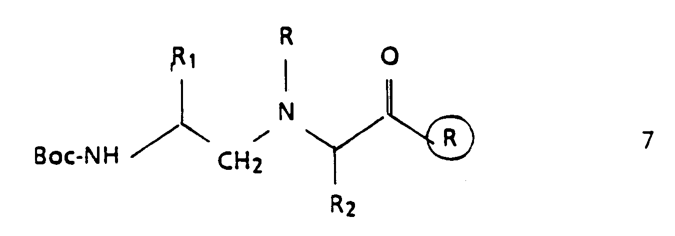
wherein R, R₁ and R₂ are as defined for formula 1 and wherein Ⓡ represents the resin.

The A₆ through A₁ amino acids can then be sequentially added to the resin bound modified dipeptide in the usual manner.

The N-methoxy-N-methyl amides of formula 2 are prepared from the corresponding N-Boc protected acid in the usual manner. Carbonyldiimidazole is added to a dried solution of the N-Boc protected amino acid in an ethereal solvent such as diethylether. The reaction mixture is allowed to stir for from 10 minutes to 1 hour typically for about 15-20 minutes. N,O-dimethylhydroxylamine HCl in DMF and a sterically hindered amine such as diisopropylethyl amine are added and the mixture allowed to stir for from about 6 hours up to about 24 hours at room temperature. The desired compound is then isolated by solvent evaporation and crude purification can be accomplished by, for example, flash chromatography on silica gel eluting with methylene chloride.

The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid.

An example of a hydroxymethyl resin is described by Bodanszky, et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitro-phenylsulfenyl, tritylsulfenyl, o-nitrophenoxyacetyl and α-chlorobutyryl; (2) aromatic urethane type protecting groups such as benzyloxycarbonyl and substituted benzyloxycarbonyl, such as p-chlorobenzyloxycarbonyl, p-nitrobenzyloxycarbonyl, p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl and allyloxycarbonyl; (4) cycloalkyl urethane type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asn or Arg is N,N′-diisopropylcarbodiimide and 1-hydroxybenzotriazole. The use of these reagents prevents nitrile and lactam formation. Other coupling agents are (1) carbodiimides (e.g., N,N′-dicyclohexylcarbodiimide and N-ethyl-N′-(Υ-dimethylaminopropylcarbodiimide); (2) cyanamides (e.g., N,N-dibenzylcyanamide); (3) ketenimines; (4) isoxazolium salts (e.g., N-ethyl-5-phenyl-isoxazolium-3′-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N′-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in anhydrous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The carboxylic hydroxyl group of Aspartic acid and Glutamic acid can be protected with a benzyl or cyclohexyl group. The preferred protecting group is benzyl.

These groups can be removed by procedures well known in the art. Typically protecting group removal is done after the peptide chain synthesis is complete but the protecting groups can be removed at any other appropriate time.

The ability of the peptide derivatives of formula 1 to act as antagonists of neurokinin A can be demonstrated by the ability of such peptides to complete with iodinated neurokinin A for mammalian neurokinin A (NK2) receptors using the method of Buck, et al., Science 226: 987-989, 1984, by the ability of such compounds to stimulate or to inhibit neurokinin A-induced phosphatidylinositol turnover using the method of Bristow, et al., British J. Pharmacol. 90: 211-21, 1987, or to antagonize neurokinin A-induced smooth muscle contraction using the method of Dion, et al., Life Sciences 41: 2269-2278, 1987.

By virtue of the ability of the peptide derivatives of this invention to act as antagonists of neurokinin A, the compounds are useful as immunosuppressants and in the treatment of arthritis, asthma, pain, inflammation, tumor growth, gastrointestinal hypermotility, Huntington's disease, psychosis, neuritis, neuralgia, headache including migraine, hypertension, urinary incontinence, urticaria, carcinoid syndrome symptoms, influenza, and common cold. Effective doses, whether oral or parenteral, can be readily determined by those of ordinary skill in the art and are those doses which cause antagonism of the neurokinin A (NK2) receptor. For example, effective doses of the peptides of this invention could be from about 0.5 »g/kg to about 500 mg/kg of the patient body weight per day. The compounds are conveniently administered in unit dosage forms containing from about 1 mg to about 500 mg of the active compound and can be administered in from one to four or more unit dosage forms per day. The term "patient" used herein is taken to mean mammals such as primates, including humans, sheep, horses, cattle, pigs, dogs, cats, rats, and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

The figures show:

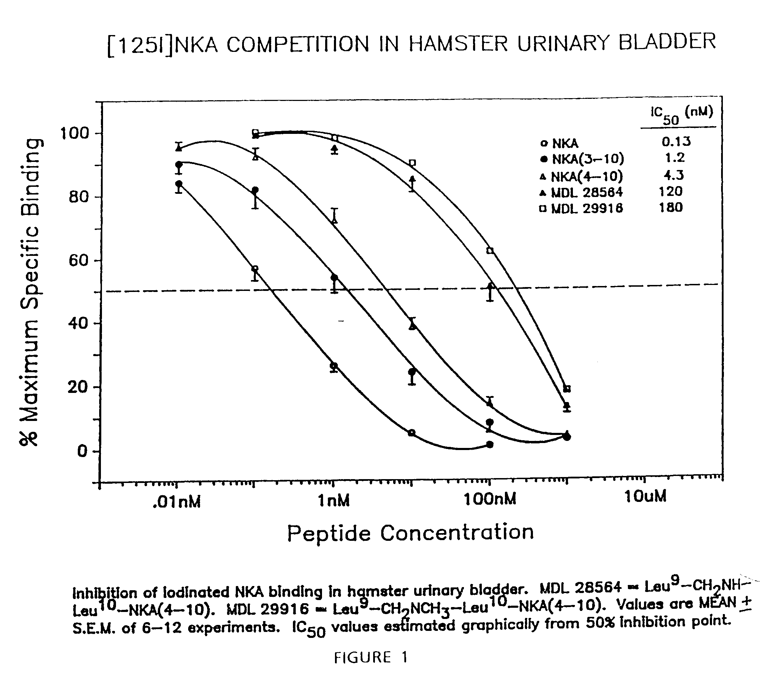

**FIGURE 1** illustrates the ability of H-Asp-Ser-Phe-Val-Gly-Leuψ[CH₂NH]Leu-NH₂ and H-Asp-Ser-Phe-Val-Gly-Leuψ[CH₂NCH₃]Leu-NH₂ to antagonize binding at the NKA receptor as demonstrated by the ability of test compound to displace ¹²⁵I labeled NKA from hamster urinary bladder (Example 1). The abscissa (x-axis) logarithmically indicates the concentration in nanomoles of agonist or antagonist of the neurokinin A (NKA) receptor. The ordinate (y-axis) indicates the observed specific binding for each tested agonist or antagonist measured as a percentage of maximum specific binding. 
NKA
NKA(3-10)
NKA (4-10)
H-Asp-Ser-Phe-Val-Gly-Leuψ[CH2NH]Leu-NH2
H-Asp-Ser-Phe-Val-Gly-Leuψ[CH2N(CH3)]Leu-NH2
 Agonists tested were NKA and fragments of NKA consisting of the third to tenth (NKA(3-10)) amino acids and the fourth to tenth (NKA(4-10)) amino acids. Values are MEAN ± S.E.M. of 6 - 12 experiments. IC₅₀ values estimated graphically from 50% inhibition point.

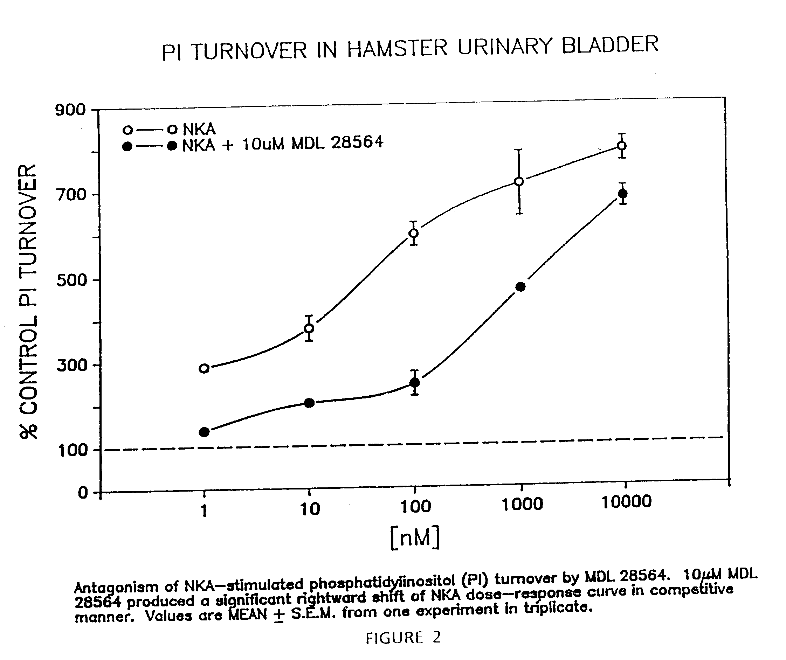

**FIGURE 2** illustrates the ability of H-Asp-Ser-Phe-Val-Gly-Leuψ[CH₂NH]Leu-NH₂ to antagonize NKA receptor binding as demonstrated by the effect on phosphatidylinositol (PI) turnover in hamster urinary bladder (Example 2). The abscissa (x-axis) logarithmically indicates the concentration in nanomolar (nM) of agonist or antagonist of the NKA receptor. The ordinate (y-axis) indicates the observed PI turnover as a percentage of control. 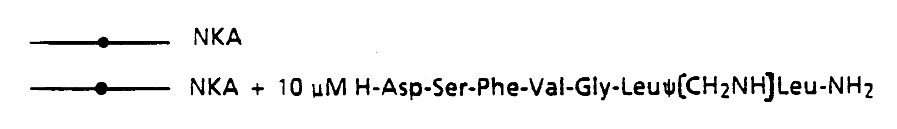
 NKA
 NKA + 10 µM H-Asp-Ser-Phe-Val-Gly-Leuψ[CH2NH]Leu-NH2
 10»M H-Asp-Ser-Phe-Val-Gly-Leuψ[CH₂NH]Leu-NH₂ produced a significant rightward shift of NKA dose-response curve in competitive manner. Values are MEAN ± S.E.M. from one experiment in triplicate.

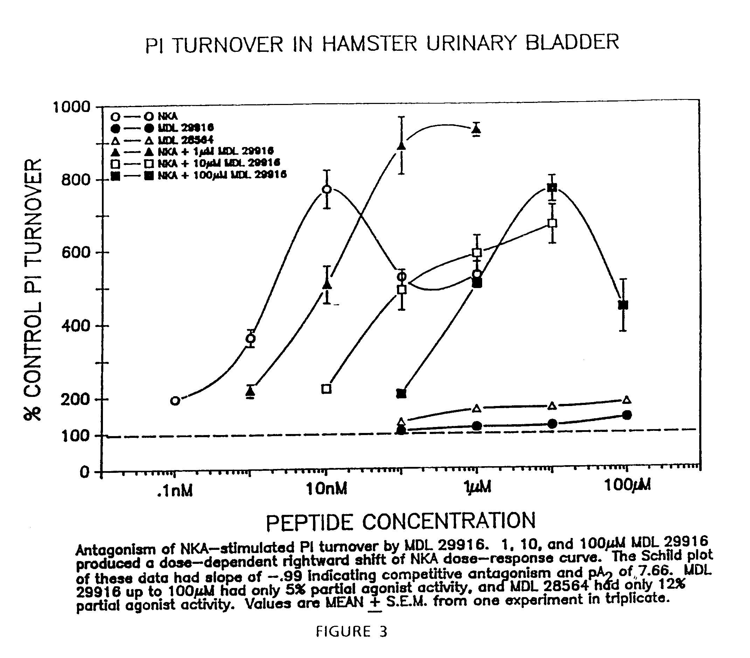

**FIGURE 3** illustrates the ability of H-Asp-Ser-Phe-Val-Gly-Leuψ[CH₂NCH₃]Leu-NH₂ (MDL 29916) to antagonize NKA receptor binding as demonstrated by the effect on phosphatidylinositol (PI) turnover in hamster urinary bladder (Example 2). The abscissa (x-axis) logarithmically indicates the concentration in nanomolar (nM) of agonist or antagonist of the NKA receptor. The ordinate (y-axis) indicates the observed PI turnover as a percentage of control. 
NKA
H-Asp-Ser-Phe-Val-Gly-Leuψ[CH2N(CH3)]Leu-NH2
H-Asp-Ser-Phe-Val-Gly-Leuψ[CH2NH]Leu-NH2
NKA + 1 μM H-Asp-Ser-Phe-Val-Gly-Leuψ[CH2N(CH3)]Leu-NH2
NKA + 10 μM H-Asp-Ser-Phe-Val-Gly-Leuψ[CH2N(CH3)]Leu-NH2
NKA + 100 μM H-Asp-Ser-Phe-Val-Gly-Leuψ[CH2N(CH3)]Leu-NH2
 1, 10 and 100 »M H-Asp-Ser-Phe-Val-Gly-Leuψ[CH₂N(CH₃)]Leu-NH₂ produced a significant rightward shift of NKA dose-response curve in competitive manner. The Schild plot of these data had slope of -.99 indicating competitive antagonism and pA2 of 7.66. H-Asp-Ser-Phe-Val-Gly-Leuψ[CH₂N(CH₃)]Leu-NH₂ up to 100»M had only 5% partial agonist activity, and H-Asp-Ser-Phe-Val-Gly-Leuψ[CH₂NH]Leu-NH₂ had only 12% partial agonist activity. Values are MEAN ± S.E.M. from one experiment in triplicate.

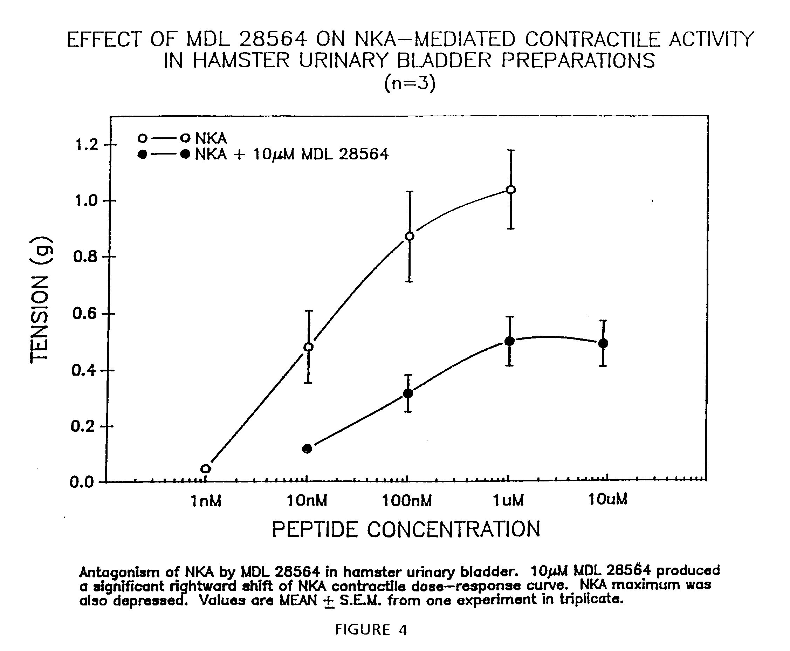

**FIGURE 4** illustrates the antagonistic effect of H-Asp-Ser-Phe-Val-Gly-Leuψ[CH₂NH]Leu-NH₂ on NKA-mediated contractile activity in hamster urinary bladder preparations (Example 3). The abscissa (x-axis) logarithmically indicates the concentration in nanomolar (nM) of NKA or NKA with 10 »M of H-Asp-Ser-Phe-Val-Gly-Leuψ[CH₂NH]Leu-NH₂. Values are MEAN ± S.E.M. from one experiment in triplicate.

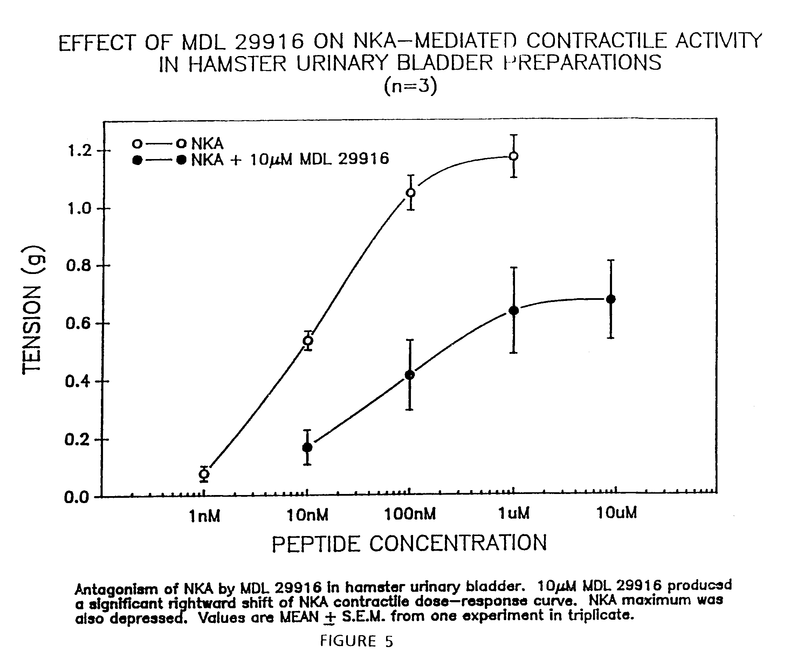

**FIGURE 5** illustrates the antagonistic effect of H-Asp-Ser-Phe-Val-Gly-Leuψ[CH₂NCH₃]Leu-NH₂ on NKA-mediated contractile activity in hamster urinary bladder preparations (Example 3). The abscissa (x-axis) logarithmically indicates the concentration in nanomolar (nM) of NKA or NKA with 10 »M of H-Asp-Ser-Phe-Val-Gly-Leuψ[CH₂N(CH₃)]Leu-NH₂. Values are MEAN ± S.E.M. from one experiment in triplicate.

### EXAMPLES

This invention is illustrated by the following, nonlimiting examples.

### EXAMPLE 1

### ANTAGONISM OF THE NEUROKININ A RECEPTOR BY H-ASP-SER-PHE-VAL-GLY-LEU-Ψ[CH₂NH]-LEU-NH₂ and H-ASP-SER-PHE-VAL-GLY-LEU-Ψ[CH₂N(CH₃)]-LEU-NH₂ AS DEMONSTRATED BY their ability to displace ¹²⁵I labeled NKA

Urinary bladders from several hamsters were pooled, minced, and homogenized in 50 mM TRIS-HCl (pH 7.4) containing 120 mM NaCl and 5 mM KCl at 4°C and centrifuged at 48,000 X g for 15 minutes. The pellet was resuspended for 30 minutes in 50 mM TRIS-HCl (pH 7.4) containing 10 mM EDTA and 300 mM KCl at 4°C. The suspension was centrifuged as above and the pellet was washed two times in plain 50 mM TRIS-HCl (pH 7.4) and centrifuged similarly. The tissue was then resuspended in incubation buffer and an aliquot (approximately 3-5 mg tissue) was added to each assay tube to commence the assay. The assay tubes contained incubation buffer consisting of 50 mM TRIS-HCl (pH 7.4), 0.02% BSA, 40 »g/ml bacitracin, 4 »g/ml chymostatin, 4 »g/ml leupeptin, 2 mM MnCl₂, 0.1 nM ¹²⁵iodohistidyl-neurokinin A (Amersham Corp.), and concentrations of the title compounds or standards ranging from 0.03 nM to 100 »M. The assay was allowed to proceed to equilibrium for 120 min at room temperature. After this time, the contents of each tube was rapidly filtered over Whatman GF/B filters presoaked in 0.5% BSA and the filters were rapidly washed two times with ice-cold plain 50 mM TRIS-HCl (pH 7.4). Filter-bound radioactivity was quantitated in a gamma counter. Specific binding (maximum) was defined as the difference between binding in the presence and absence of 1 »M unlabeled neurokinin A. Competition of iodinated neurokinin A binding by test compounds or standards was expressed as a percentage of this maximum competition. IC₅₀ values (concentration required to inhibit 50% of receptor binding) were found to be 100-200 nM for the title compounds. (Figure 1)

### EXAMPLE 2

### ANTAGONISM OF THE NEUROKININ A RECEPTOR BY H-ASP-SER-PHE-VAL-. GLY-LEU-Ψ[CH₂NH]-LEU-NH₂ and H-ASP-SER-PHE-VAL-GLY-LEU-Ψ[CH₂N)CH₃)]-LEU-NH₂ AS DEMONSTRATED BY THE EFFECT ON PHOSPHATIDYLINOSITOL TURNOVER

Urinary bladders from several hamsters were pooled and chopped at 350 »m with a tissue chopper. The chopped tissue was then incubated in 37°C Krebs-Hepes buffer with fresh buffer changes every 15 minutes for 30 minutes. The tissue was then incubated in this buffer containing 100-200 »Ci of ³H-inositol at 37°C. The tissue was then washed and incubated for another 30 minutes in Krebs-Hepes (containing 10 mM Li⁺) at 37°C with fresh buffer change every 15 minutes. Portions of the tissue mass (approximately 10-20 mg per assay tube) were then placed in Li⁺ buffer, test compound was then added in 25 »l, and then various concentrations of neurokinin A were added in 25 »l in a final volume of 250 »l. Test compound was evaluated at concentrations ranging from 1 nM to 100 »M and neurokinin A concentrations ranged from 1 nM to 10 »M. Test compound was also evaluated alone at the indicated concentrations to test for agonist activity. After 30 minutes at room temperature, the phosphatidylinositol turnover was terminated by the addition of 940 »l chloroform:methanol (1:2), followed by 310 »l chloroform, followed by 310 »l water. Each tube was then vortexed for 15 seconds and then centrifuged at 3000 rpm for 10 minutes to separate the phases. 900 »l of the top (aqueous) phase was then loaded onto a 0.5 ml Biorad AG-1X8 (formate) ion exchange column. 50 »l of the bottom phase (chloroform) was withdrawn from each tube and placed in a counting vial, dried, and counted in scintillation fluid. The material on the columns was washed in order with:
1) 10 ml of water
2) 5 ml of 5 mM disodium tetraborate/60 mM sodium formate
3) 10 ml of 1 M ammonium formate in 0.1 M formic acid
The final (third) wash was collected and one ml was mixed with 6 ml of ACS scintillant and counted. The ratio of these counts (total inositol phosphates) to the corresponding organic phase counts was then calculated for each sample. The ratios in the presence of test compound and/or standards were then compared to the ratios for control tubes (i.e., no stimulating agonist). Dose-response curves were constructed and the abilities of test compounds to stimulate or to inhibit neurokinin A-induced phosphatidylinositol turnover were determined by graphical analysis or with the aid of a computer program and are illustrated in Figures 2 and 3.

### EXAMPLE 3

### HAMSTER URINARY BLADDER CONTRACTION PREPARATION

Half-strips of urinary bladders from male golden Syrian hamsters (75 - 100 g) were suspended in Tyrode's buffer at 31°C and with 1 gram resting tension as described by Dion et al. (Life Sciences 41: 2269 - 2278, 1987). The enkephalinase inhibitor thiorphan was added to the buffer at 10 »M 15 minutes prior to each test compound addition. Cumulative NKA dose-response curves were constructed first in the absence and then in the presence of test compound. The test compounds were added cumulatively as well to ascertain whether or not they had any contractile effects themselves. Any test compound effect that was observed was allowed to plateau before the next cumulative concentration was added. Under these conditions, the EC₅₀ for NKA contractile effects was generally 10 nM, in good agreement with literature values. Contractile data are expressed as grams of tension developed over and above the resting tension. In three separate hamster bladder tissues each, H-Asp-Ser-Phe-Val-Gly-Leuψ[CH₂NH]Leu-NH₂ or Asp-Ser-Phe-Val-Gly-Leuψ[CH₂N(CH₃)]Leu-NH₂ up to concentrations of 1 »M did not produce any contraction. Figures 4 and 5 illustrate the contractile force as a function of NKA concentration in the absence and presence of test compound.

### EXAMPLE 4

### I. Boc-Leu-aldehyde synthesis (Fehrentz, J.-A. and Castro, B Synthesis, 1983, 676-678):

### A. N-t-Boc-Leucine N-methoxy-N-methylamide:

15.0 mmoles of Boc-Leucine hydrate was dissolved in 30 ml dry ether. The solution was dried over anhydrous MgSO₄ and the solid removed by filtration. 16.5 mmoles of carbonyldiimidazole was added to the filtrate and the reaction mixture stirred 20 minutes at room temperature. To the resulting solution was added a suspension of 0,N-dimethylhydroxylamine hydrochloride (22.5 mmoles) in 15 ml dimethylformamide and 3.9 ml diisopropylethylamine. The reaction mixture was stirred overnight at room temperature. The reaction mixture was diluted into ethyl acetate (75 ml) and washed with cold 1N HCl (3 x 40 ml), saturated NaHCO₃ (3 x 40 ml) and saturated NaCl (1 x 40 ml). The organic phase was dried with MgSO₄, filtered and the ethyl acetate removed *in vacuo*. Analytical Data: R_{f} (silica gel F254): 0.51 (ethyl acetate/hexane 3/2); 'H-NMR (CDCl₃)(TMS int): δ=0.95 ppm (2d, 6H, J=6.6 Hz); 1.42 (s, 11H); 1.64-1.80 (m, 1H); 3.20 (s, 3H); 3.80 (s,3H); 4.72 (m, 1H); 5.10 (d, 1H, J=7.5 Hz). Mass Spec: M+H⁺: Theoretical: 275. Observed: 275.

### B. Boc-Leucine aldehyde:

2.5 mmoles of Boc-leucine N-methoxy-N-methylamide were dissolved in dry ether (30 ml). To this solution was added 3.2 mmole of lithium aluminum hydride (1M solution in tetrahydrofuran). The reaction was stirred 20 minutes at room temperature and then carefully quenched by addition of a solution of NaHSO₄ (0.6 g) in 10 ml water. The reaction mixture was added to 75 ml ether and washed with cold 1N HCl (3 x 30 ml), saturated NaHCO₃ (3 x 30 ml) and saturated NaCl (1 x 30 ml). The organic phase was dried with MgSO₄, filtered and the solvent removed *in vacuo*. Analytical Data: Rf (silica gel 60 F254): 0.68 (ethyl acetate/hexane 3/2). Mass Spec: M+H⁺: Theoretical: 216. Observed: 216.

### II. Synthesis of Leu-Resin:

Standard solid phase peptide synthesis techniques on an automated peptide synthesizer were used. After formation of Boc-Leu-resin (0.5 mmoles), the Boc protecting group was removed and the resin washed with dimethylformamide, dichloromethane and dried.

### III. Formation of reduced amide bond (Leuψ[CH₂NH]):

The Boc-Leu aldehyde (2 mmoles) was dissolved in 1% acetic acid in dimethylformamide (10 ml) and this solution was added to the reaction vessel containing the Leu-resin (0.5 mmoles, see II. above). To this mixture was added a solution of NaCNBH₃ (150 mg) in 2 ml dimethylformamide. The mixture was shaken for 4 hours, the reaction vessel was drained and the resin washed with dimethylformamide, then dichloromethane.

### IV. Synthesis of [ψ[CH₂NH]⁹, Leu¹⁰]NKA₄₋₁₀:

The synthesis of the peptide was completed on the automated peptide synthesizer by sequential addition of the remaining amino acids (Gly, Val, Phe, Ser (Bzl) and Asp (Chxl)). The peptide was cleaved from the resin and globally deprotected using anhydrous HF/anisole (10:1). The peptide was purified using reverse phase HPLC techniques. Analytical Data: Amino Acid Analysis: (HCl digest) Asp (1.03); Ser (0.93); Gly (1.01); Val (0.96); Phe (0.74). Peptide Content: 53.4%. Fast Atom Bombardment Mass Spectrometry: M+H⁺ Theoretical: 735. Observed: 735.

### V. Synthesis of [ψ[CH₂NCH₃]⁹, LEU¹⁰]NKA₄₋₁₀:

N-Methyl-Leu-resin (0.5 mmoles) (prepared from Boc-N-methyl Leucine according to II above) was reacted with 2.0 mmoles Boc-Leu aldehyde as described in III above. The peptide synthesis was then completed as described in IV above. Analytical Data: Amino Acid Analysis: (HCl digest) Asp (1.01); Ser (0.89); Gly (1.02); Val (1.00); Phe (0.98). Peptide Content: 53.5%. Fast Atom Bombardment Mass Spectrometry: M+H⁺. Theoretical: 749. Observed: 749.

### VI. Synthesis of [ψ[CH₂NCH₂R]⁹, Leu¹⁰]NKA₄₋₁₀:

Boc-Leu [ψ(CH₂NH)]-Leu resin is prepared according to steps II and III above. The resin is then reacted with 2.5 mmoles of R-CHO in 10 ml of 1% HOAc in dimethylformamide in the presence of NaCNBH₃ as described in III above. The synthesis of the peptide is then completed as described in IV above.

## Claims (Claims for the following Contracting State(s): AT, BE, CH, DE, FR, GB, IT, LI, LU, NL, SE)

1. A peptide derivative of the general formula I
X-A₁-A₂-A₃-A₄-A₅-A₆-Y (I)
wherein
X is hydrogen, an alkyl group of from 1 to 6 carbon atoms, or an acyl group of from 2 to 10 carbon atoms;
A₁ is a bond or His-Lys-Thr, Lys-Thr, Thr, Val-Pro-Lys-Ser, Pro-Lys-Ser, Lys-Ser, Ser, pGlu-Pro-Ser-Lys, Pro-Ser- Lys, Ser-Lys, Lys, or His;
A₂ is a bond or is Asp or Glu;
A₃ is Gly, Gln, Asn, Sar, Ala, or Ser;
A₄ is Phe or N-Me-Phe;
A₅ is Ile, Val, Leu, Phe, Ala, Tyr, Nle, Met, or N-Me-Val;
A₆ is Gly or Sar; and
Y is a group of the formula 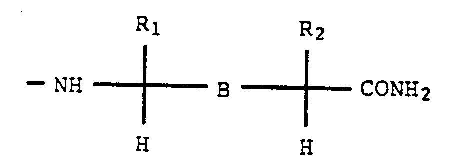 wherein
B is a group of one of the formulae 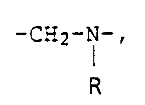 -CH₂-S-,
-CH₂-O-,
-CH=CH-, 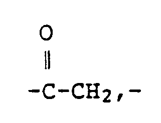
-CH(OH)CH₂-, or 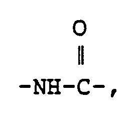 and wherein
R is a hydrogen atom or an alkyl group of from 1 to 4 carbon atoms or is a phenylalkylene group wherein the alkylene moiety is straight or branched and has from 1 to 6 carbon atoms and wherein the phenyl moiety is unsubstituted or is mono substituted with a C₁₋₄ alkyl, C₁₋₄ alkoxy, hydroxy, or halogen group;
R₁ and R₂ are each independently selected from isopropyl, isobutyl, sec-butyl, n-butyl, and 2-(methylthio)ethyl groups
or a pharmaceutically acceptable salt thereof.

2. The peptide derivative of claim 1 wherein X is hydrogen.

3. The peptide derivative of claim 1 or 2 wherein A₁ is a bond and X is Glt, Mal, Fum, or Suc.

4. The peptide derivative of any one of claims 1 to 3 wherein A₁ is a bond and X is Suc.

5. The peptide derivative of claim 1 wherein A₁ is His-Lys-Thr, Lys-Thr, or Thr.

6. The peptide derivative of any one of claims 1 to 5 wherein A₂ is Asp.

7. The peptide derivative of any one of claims 1 to 6 wherein A₃ is Ala, or Ser.

8. The peptide derivative of any one of claims 1 to 7 wherein A₄ is Phe.

9. The peptide derivative of any one of claims 1 to 8 wherein A₅ is Val.

10. The peptide derivative of any one of claims 1 to 9 wherein A₆ is Gly.

11. The peptide derivative of any one of claims 1 to 10 wherein B is

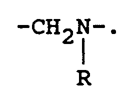

12. The peptide derivative of any one of claims 1 to 11 wherein R₁ is isobutyl.

13. The peptide derivative of any one of claims 1 to 12 wherein R₂ is 2-methylthioethyl, isobutyl, or n-butyl.

14. The peptide derivative of any one of claims 1 to 13 wherein R₁ and R₂ are each an isobutyl.

15. The peptide derivative of claim 1 wherein X is hydrogen, Glt, Mal, Fum, or Suc; A₁ is a bond, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, or Lys; A₂ is a bond, Asp, or Glu; A₃ is Gly, Gln, Asn, Sar, Ala, or Ser; A₄ is Phe or N-Me-Phe; A₅ is Ile, Val, Leu, Phe, Ala, Tyr, Nle, Met, or N-Me-Val; A₆ is Gly or Sar; B is -CH₂NH-; and R₁ and R₂ are each independently selected from isopropyl, isobutyl, sec-butyl, n-butyl, or 2-(methylthio)ethyl.

16. The peptide derivative of claim 15 wherein X is hydrogen.

17. The peptide derivative of claim 15 or 16 wherein A₁ is a bond, His-Lys-Thr, Lys-Thr, or Thr.

18. The peptide derivative of any one of claims 15 to 17 wherein A₂ is a bond or Asp.

19. The peptide derivative of any one of claims 15 to 18 wherein X is hydrogen; A₁ is His-Lys-Thr, Lys-Thr, or Thr; and A₂ is Asp.

20. The peptide derivative of any one of claims 15 to 19 wherein A₃ is Ala or Ser.

21. The peptide derivative of any one of claims 15 to 20 wherein A₄ is Phe.

22. The peptide derivative of any one of claims 15 to 21 wherein A₅ is Val.

23. The peptide derivative of any one of claims 15 to 22 wherein A₆ is Gly.

24. The peptide derivative of any one of claims 15 to 23 wherein B is

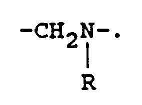

25. The peptide derivative of any one of claims 15 to 24 wherein R₁ is sec-butyl.

26. The peptide derivative of any one of claims 15 to 25 wherein R₂ is 2-(methylthio)ethyl, sec-butyl, or n-butyl.

27. The peptide derivative of claim 15 which is H-Asp-Ser-Phe-Val-Gly-LeuΨ[CH₂NH]Leu-NH₂ or H-Asp-Ser-Phe-Val-Gly-LeuΨ[CH₂N(CH₃)]Leu-NH₂.

28. A process for preparing a peptide derivative according to any one of claims 1 to 27 or a pharmaceutically acceptable salt thereof which comprises binding to a resin bound compound of the formula 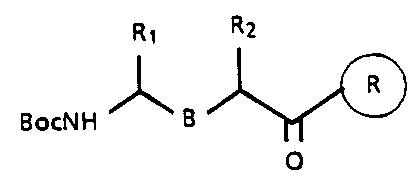 wherein R₁, R₂, and B are as defined in the preceding claims and wherein Boc is a butyloxycarbonyl protecting group and R (with the circle around it) represents the resin the alpha amino protected amino acids in order from A₆ through A₁ to the terminal amino group of the growing peptidic chain which has meanwhile been exposed by removing its amino protecting group.

29. A peptide derivative of any one of claims 1 to 27 or a mixture thereof for use as a pharmaceutically active substance.

30. A peptide derivative of any one of claims 1 to 27 or a mixture thereof for use as a neurokinin A antagonist.

31. A pharmaceutical composition for the treatment of asthma, inflammations and/or arthritis containing an effective amount of a peptide derivative of any one of claims 1 to 27 or of a mixture thereof and optionally a pharmaceutically acceptable carrier and/or diluent.

## Claims (Claims for the following Contracting State(s): ES)

1. A process for preparing peptide derivatives of the general formula I
X-A₁-A₂-A₃-A₄-A₅-A₆-Y (I)
wherein
X is hydrogen, an alkyl group of from 1 to 6 carbon atoms, or an acyl group of from 2 to 10 carbon atoms;
A₁ is a bond or His-Lys-Thr, Lys-Thr, Thr, Val-Pro-Lys-Ser, Pro-Lys-Ser, Lys-Ser, Ser, pGlu-Pro-Ser-Lys, Pro-Ser- Lys, Ser-Lys, Lys, or His;
A₂ is a bond or is Asp or Glu;
A₃ is Gly, Gln, Asn, Sar, Ala, or Ser;
A₄ is Phe or N-Me-Phe;
A₅ is Ile, Val, Leu, Phe, Ala, Tyr, Nle, Met, or N-Me-Val;
A₆ is Gly or Sar; and
Y is a group of the formula 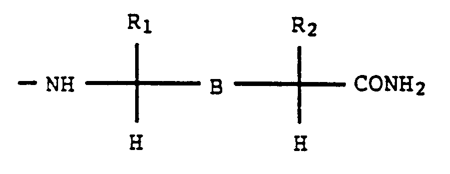 wherein
B is a group of one of the formulae 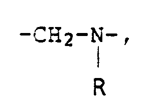 -CH₂-S-,
-CH₂-O-,
-CH=CH-, 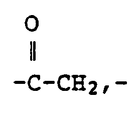 -CH(OH)CH₂-, or 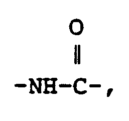 and wherein
R is a hydrogen atom or an alkyl group of from 1 to 4 carbon atoms or is a phenylalkylene group wherein the alkylene moiety is straight or branched and has from 1 to 6 carbon atoms and wherein the phenyl moiety is unsubstituted or is mono substituted with a C₁₋₄ alkyl, C₁₋₄ alkoxy, hydroxy, or halogen group;
R₁ and R₂ are each independently selected from isopropyl, isobutyl, sec-butyl, n-butyl, and 2-(methylthio)ethyl groups
or a pharmaceutically acceptable salt thereof, which comprises binding to a resin bound compound of the formula 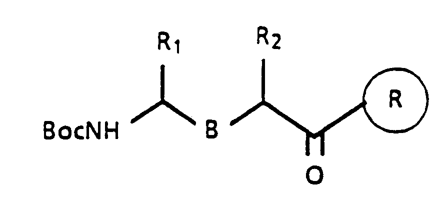 wherein R₁, R₂, and B are as defined above and wherein Boc is a butyloxycarbonyl protecting group and R (with the circle around it) represents the resin the alpha amino protected amino acids in order from A₆ through A₁ to the terminal amino group of the growing peptidic chain which has meanwhile been exposed by removing its amino protecting group.

2. The process according to claim 1 wherein X is hydrogen.

3. The process according to claim 1 or 2 wherein A₁ is a bond and X is Glt, Mal, Fum, or Suc.

4. The process according to any one of claims 1 to 3 wherein A₁ is a bond and X is Suc.

5. The process according to claim 1 wherein A₁ is His-Lys-Thr, Lys-Thr, or Thr.

6. The process according to any one of claims 1 to 5 wherein A₂ is Asp.

7. The process according to any one of claims 1 to 6 wherein A₃ is Ala, or Ser.

8. The process according to any one of claims 1 to 7 wherein A₄ is Phe.

9. The process according to any one of claims 1 to 8 wherein A₅ is Val.

10. The process according to any one of claims 1 to 9 wherein A₆ is Gly.

11. The process according to any one of claims 1 to 10 wherein B is

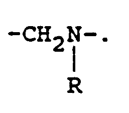

12. The process according to any one of claims 1 to 11 wherein R₁ is isobutyl.

13. The process according to any one of claims 1 to 12 wherein R₂ is 2-methylthioethyl, isobutyl, or n-butyl.

14. The process according to any one of claims 1 to 13 wherein R₁ and R₂ are each an isobutyl.

15. The process according to claim 1 wherein X is hydrogen, Glt, Mal, Fum, or Suc; A₁ is a bond, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, or Lys; A₂ is a bond, Asp, or Glu; A₃ is Gly, Gln, Asn, Sar, Ala, or Ser; A₄ is Phe or N-Me-Phe; A₅ is Ile, Val, Leu, Phe, Ala, Tyr, Nle, Met, or N-Me-Val; A₆ is Gly or Sar; B is -CH₂NH-; and R₁ and R₂ are each independently selected from isopropyl, isobutyl, sec-butyl, n-butyl, or 2-(methylthio)ethyl.

16. The process according to claim 15 wherein X is hydrogen.

17. The process according to claim 15 or 16 wherein A₁ is a bond, His-Lys-Thr, Lys-Thr, or Thr.

18. The process according to any one of claims 15 to 17 wherein A₂ is a bond or Asp.

19. The process according to any one of claims 15 to 18 wherein X is hydrogen; A₁ is His-Lys-Thr, Lys-Thr, or Thr; and A₂ is Asp.

20. The process according to any one of claims 15 to 19 wherein A₃ is Ala or Ser.

21. The process according to any one of claims 15 to 20 wherein A₄ is Phe.

22. The process according to any one of claims 15 to 21 wherein A₅ is Val.

23. The process according to any one of claims 15 to 22 wherein A₆ is Gly.

24. The process according to any one of claims 15 to 23 wherein B is

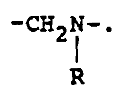

25. The process according to any one of claims 15 to 24 wherein R₁ is sec-butyl.

26. The process according to any one of claims 15 to 25 wherein R₂ is 2-(methylthio)ethyl, sec-butyl, or n-butyl.

27. The process according to claim 15 wherein the compound obtained is H-Asp-Ser-Phe-Val-Gly-LeuΨ[CH₂NH]Leu-NH₂ or H-Asp-Ser-Phe-Val-Gly-LeuΨ[CH₂N(CH₃)]Leu-NH₂.

28. Use of a peptide derivative obtainable according to a process of any one of claims 1 to 27 for the preparation of a pharmaceutical composition.

29. Use of a peptide derivative obtainable according to a process of any one of claims 1 to 27 for the preparation of a pharmaceutical composition for the treatment of asthma, inflammations and/or arthritis.

## Claims (Claims for the following Contracting State(s): GR)

1. A peptide derivative of the general formula I
X-A₁-A₂-A₃-A₄-A₅-A₆-Y (I)
wherein
X is hydrogen, an alkyl group of from 1 to 6 carbon atoms, or an acyl group of from 2 to 10 carbon atoms;
A₁ is a bond or His-Lys-Thr, Lys-Thr, Thr, Val-Pro-Lys-Ser, Pro-Lys-Ser, Lys-Ser, Ser, pGlu-Pro-Ser-Lys, Pro-Ser- Lys, Ser-Lys, Lys, or His;
A₂ is a bond or is Asp or Glu;
A₃ is Gly, Gln, Asn, Sar, Ala, or Ser;
A₄ is Phe or N-Me-Phe;
A₅ is Ile, Val, Leu, Phe, Ala, Tyr, Nle, Met, or N-Me-Val;
A₆ is Gly or Sar; and
Y is a group of the formula 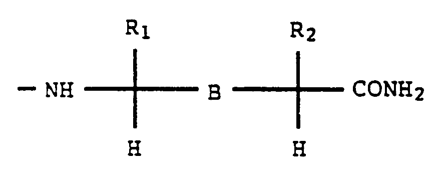 wherein
B is a group of one of the formulae 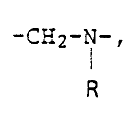 -CH₂-S-,
-CH₂-O-,
-CH=CH-, 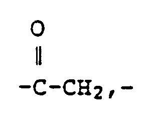 -CH(OH)CH₂-, or 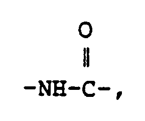 and wherein
R is a hydrogen atom or an alkyl group of from 1 to 4 carbon atoms or is a phenylalkylene group wherein the alkylene moiety is straight or branched and has from 1 to 6 carbon atoms and wherein the phenyl moiety is unsubstituted or is mono substituted with a C₁₋₄ alkyl, C₁₋₄ alkoxy, hydroxy, or halogen group;
R₁ and R₂ are each independently selected from isopropyl, isobutyl, sec-butyl, n-butyl, and 2-(methylthio)ethyl groups
or a pharmaceutically acceptable salt thereof.

2. The peptide derivative of claim 1 wherein X is hydrogen.

3. The peptide derivative of claim 1 or 2 wherein A₁ is a bond and X is Glt, Mal, Fum, or Suc.

4. The peptide derivative of any one of claims 1 to 3 wherein A₁ is a bond and X is Suc.

5. The peptide derivative of claim 1 wherein A₁ is His-Lys-Thr, Lys-Thr, or Thr.

6. The peptide derivative of any one of claims 1 to 5 wherein A₂ is Asp.

7. The peptide derivative of any one of claims 1 to 6 wherein A₃ is Ala, or Ser.

8. The peptide derivative of any one of claims 1 to 7 wherein A₄ is Phe.

9. The peptide derivative of any one of claims 1 to 8 wherein A₅ is Val.

10. The peptide derivative of any one of claims 1 to 9 wherein A₆ is Gly.

11. The peptide derivative of any one of claims 1 to 10 wherein B is

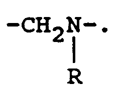

12. The peptide derivative of any one of claims 1 to 11 wherein R₁ is isobutyl.

13. The peptide derivative of any one of claims 1 to 12 wherein R₂ is 2-methylthioethyl, isobutyl, or n-butyl.

14. The peptide derivative of any one of claims 1 to 13 wherein R₁ and R₂ are each an isobutyl.

15. The peptide derivative of claim 1 wherein X is hydrogen, Glt, Mal, Fum, or Suc; A₁ is a bond, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, or Lys; A₂ is a bond, Asp, or Glu; A₃ is Gly, Gln, Asn, Sar, Ala, or Ser; A₄ is Phe or N-Me-Phe; A₅ is Ile, Val, Leu, Phe, Ala, Tyr, Nle, Met, or N-Me-Val; A₆ is Gly or Sar, B is -CH₂NH-; and R₁ and R₂ are each independently selected from isopropyl, isobutyl, sec-butyl, n-butyl, or 2-(methylthio)ethyl.

16. The peptide derivative of claim 15 wherein X is hydrogen.

17. The peptide derivative of claim 15 or 16 wherein A₁ is a bond, His-Lys-Thr, Lys-Thr, or Thr.

18. The peptide derivative of any one of claims 15 to 17 wherein A₂ is a bond or Asp.

19. The peptide derivative of any one of claims 15 to 18 wherein X is hydrogen; A₁ is His-Lys-Thr, Lys-Thr, or Thr; and A₂ is Asp.

20. The peptide derivative of any one of claims 15 to 19 wherein A₃ is Ala or Ser.

21. The peptide derivative of any one of claims 15 to 20 wherein A₄ is Phe.

22. The peptide derivative of any one of claims 15 to 21 wherein A₅ is Val.

23. The peptide derivative of any one of claims 15 to 22 wherein A₆ is Gly.

24. The peptide derivative of any one of claims 15 to 23 wherein B is

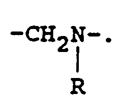

25. The peptide derivative of any one of claims 15 to 24 wherein R₁ is sec-butyl.

26. The peptide derivative of any one of claims 15 to 25 wherein R₂ is 2-(methylthio)ethyl, sec-butyl, or n-butyl.

27. The peptide derivative of claim 15 which is H-Asp-Ser-Phe-Val-Gly-LeuΨ[CH₂NH]Leu-NH₂ or H-Asp-Ser-Phe-Val-Gly-LeuΨ[CH₂N(CH₃)]Leu-NH₂.

28. A process for preparing a peptide derivative according to any one of claims 1 to 27 or a pharmaceutically acceptable salt thereof which comprises binding to a resin bound compound of the formula 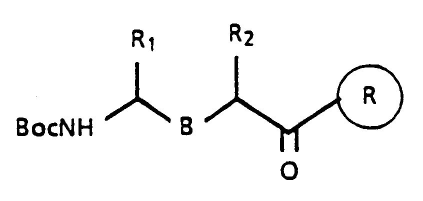 wherein R₁, R₂, and B are as defined in the preceding claims and wherein Boc is a butyloxycarbonyl protecting group and R (with the circle around it) represents the resin the alpha amino protected amino acids in order from A₆ through A₁ to the terminal amino group of the growing peptidic chain which has meanwhile been exposed by removing its amino protecting group.

29. A peptide derivative of any one of claims 1 to 27 or a mixture thereof for use as a pharmaceutically active substance.

30. A peptide derivative of any one of claims 1 to 27 or a mixture thereof for use as a neurokinin A antagonist.

## Patentansprüche (Patentansprüche für folgende(n) Vertragsstaat(en): AT, BE, CH, DE, FR, GB, IT, LI, LU, NL, SE)

1. Peptidderivat der allgemeinen Formel I
X-A₁-A₂-A₃-A₄-A₅-A₆-Y (I)
in der X ein Wasserstoffatom, einen Alkylrest mit 1 bis 6 Kohlenstoffatomen oder einen Acylrest mit 2 bis 10 Kohlenstoffatomen bedeutet,
A₁ eine Bindung oder His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys oder His bedeutet,
A₂ eine Bindung oder Asp oder Glu darstellt,
A₃ Gly, Gln, Asn, Sar, Ala oder Ser bedeutet,
A₄ Phe oder N-Me-Phe darstellt,
A₅ Ile, Val, Leu, Phe, Ala, Tyr, Nle, Met oder N-Me-Val bedeutet;
A₆ Gly oder Sar bedeutet, und
Y einen Rest der Formel 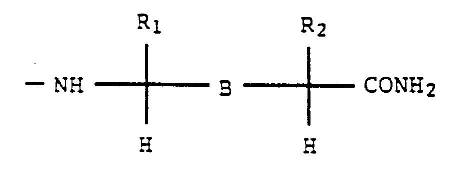 darstellt, wobei B einen Rest mit einer der folgenden Formeln darstellt 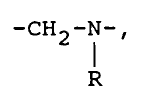 -CH₂-S-, -CH₂-O-, -CH=CH-, 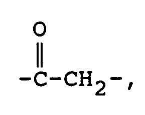 -CH(OH)CH₂-, oder 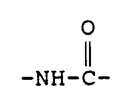 und wobei R ein Wasserstoffatom oder einen Alkylrest mit 1 bis 4 Kohlenstoffatomen oder einen Phenylalkylenrest darstellt, wobei die Alkyleneinheit geradkettig oder verzweigt ist und 1 bis 6 Kohlenstoffatome aufweist und wobei die Phenyleinheit unsubstituiert ist oder mit einem C₁₋₄-Alkylrest, C₁₋₄-Alkoxyrest, einer Hydroxylgruppe oder einem Halogenatom monosubstituiert ist,
R₁ und R₂ jeweils unabhängig voneinander ausgewählt sind aus Isopropyl-, Isobutyl-, sek.-Butyl-, n-Butyl- und 2-(Methylthio)ethylgruppen,
oder ein pharmazeutisch verträgliches Salz davon.

2. Peptidderivat nach Anspruch 1, wobei X ein Wasserstoffatom ist.

3. Peptidderivat nach Anspruch 1 oder 2, wobei A₁ eine Bindung und X Glt, Mal, Fum oder Suc bedeutet.

4. Peptidderivat nach einem der Ansprüche 1 bis 3, wobei A₁ eine Bindung und X Suc bedeutet.

5. Peptidderivat nach Anspruch 1, wobei A₁ His-Lys-Thr, Lys-Thr oder Thr bedeutet.

6. Peptidderivat nach einem der Ansprüche 1 bis 5, wobei A₂ Asp bedeutet.

7. Peptidderivat nach einem der Ansprüche 1 bis 6, wobei A₃ Ala oder Ser bedeutet.

8. Peptidderivat nach einem der Ansprüche 1 bis 7, wobei A₄ Phe bedeutet.

9. Peptidderivat nach einem der Ansprüche 1 bis 8, wobei A₅ Val bedeutet.

10. Peptidderivat nach einem der Ansprüche 1 bis 9, wobei A₆ Gly bedeutet.

11. Peptidderivat nach einem der Ansprüche 1 bis 10, wobei B 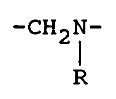 bedeutet.

12. Peptidderivat nach einem der Ansprüche 1 bis 11, wobei R₁ eine Isobutylgruppe ist.

13. Peptidderivat nach einem der Ansprüche 1 bis 12, wobei R₂ eine 2-Methylthioethyl-, Isobutyl- oder n-Butylgruppe bedeutet.

14. Peptidderivat nach einem der Ansprüche 1 bis 13, wobei R₁ und R₂ jeweils eine Isobutylgruppe bedeuten.

15. Peptidderivat nach Anspruch 1, wobei X ein Wasserstoffatom, Glt, Mal, Fum oder Suc bedeutet, A₁ eine Bindung, His-Lys-Thr, Lys-Thr, Thr, Val-Pro-Lys-Ser,Pro-Lys-Ser, Lys-Ser, Ser, pGlu-Pro-Ser-Lys, Pro-Ser-Lys, Ser-Lys oder Lys darstellt, A₂ eine Bindung, Asp oder Glu bedeutet, A₃ Gly, Gln, Asn, Sar, Ala oder Ser bedeutet, A₄ Phe oder N-Me-Phe bedeutet, A₅ Ile, Val, Leu, Phe, Ala, Tyr, Nle, Met oder N-Me-Val bedeutet, A₆ Gly oder Sar bedeutet, B -CH₂NH- bedeutet und R₁ und R₂ unabhängig voneinander ausgewählt sind aus einer Isopropyl-, Isobutyl-, sek.-Butyl-, n-Butyl- oder 2-(Methylthio)ethyl-Gruppe.

16. Peptidderivat nach Anspruch 15, wobei X ein Wasserstoffatom ist.

17. Peptidderivat nach Anspruch 15 oder 16, wobei A₁ eine Bindung, His-Lys-Thr, Lys-Thr oder Thr bedeutet.

18. Peptidderivat nach einem der Ansprüche 15 bis 17, wobei A₂ eine Bindung oder Asp bedeutet.

19. Peptidderivat nach einem der Ansprüche 15 bis 18, wobei X ein Wasserstoffatom darstellt, A₁ His-Lys-Thr, Lys-Thr oder Thr darstellt und A₂ Asp bedeutet.

20. Peptidderivat nach einem der Ansprüche 15 bis 19, wobei A₃ Ala oder Ser bedeutet.

21. Peptidderivat nach einem der Ansprüche 15 bis 20, wobei A₄ Phe bedeutet.

22. Peptidderivat nach einem der Ansprüche 15 bis 21, wobei A₅ Val bedeutet.

23. Peptidderivat nach einem der Ansprüche 15 bis 22, wobei A₆ Gly bedeutet.

24. Peptidderivat nach einem der Ansprüche 15 bis 23, wobei B 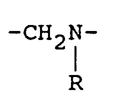 bedeutet.

25. Peptidderivat nach einem der Ansprüche 15 bis 24, wobei R₁ eine sek.-Butylgruppe ist.

26. Peptidderivat nach einem der Ansprüche 15 bis 25, wobei R₂ eine 2-(Methylthio)ethyl-, sek.-Butyl- oder n-Butylgruppe ist.

27. Peptidderivat nach Anspruch 15, nämlich
H-Asp-Ser-Phe-Val-Gly-LeuΨ[CH₂NH]Leu-NH₂ oder
H-Asp-Ser-Phe-Val-Gly-LeuΨ[CH₂N(CH₃)]Leu-NH₂.

28. Verfahren zur Herstellung eines Peptidderivats nach einem der Ansprüche 1 bis 27 oder eines pharmazeutisch verträglichen Salzes davon, umfassend die Bindung an eine harzgebundene Verbindung der Formel 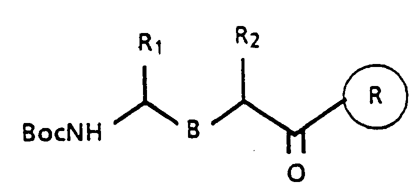 in der R₁, R₂ und B die in den vorangehenden Ansprüchen angegebene Bedeutung haben und wobei Boc eine Butyloxycarbonyl-Schutzgruppe ist und R (mit dem Kreis darum) das Harz darstellt, der α-aminogeschützten Aminosäuren in der Reihenfolge von A₆ bis zu A₁ an die terminale Aminogruppe der wachsenden Peptidkette, welche mittlerweile durch Entfernung ihrer Aminoschutzgruppe freigelegt wurde.

29. Peptidderivat nach einem der Ansprüche 1 bis 27 oder ein Gemisch davon zur Verwendung als pharmazeutisch wirksamer Stoff.

30. Peptidderivat nach einem der Ansprüche 1 bis 27 oder ein Gemisch davon zur Verwendung als Neurokinin A-Antagonist.

31. Arzneimittel für die Behandlung von Asthma, Entzündungen und/oder Arthritis, enthaltend eine wirksame Menge eines Peptidderivats nach einem der Ansprüche 1 bis 27 oder ein Gemisch davon und gegebenenfalls einen pharmazeutisch verträglichen Träger und/oder Verdünnungsmittel.

## Patentansprüche (Patentansprüche für folgende(n) Vertragsstaat(en): ES)

1. Verfahren zur Herstellung von Peptidderivaten der allgemeinen Formel I
X-A₁-A₂-A₃-A₄-A₅-A₆-Y (I)
in der X ein Wasserstoffatom, einen Alkylrest mit 1 bis 6 Kohlenstoffatomen oder einen Acylrest mit 2 bis 10 Kohlenstoffatomen bedeutet,
A₁ eine Bindung oder His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys oder His bedeutet,
A₂ eine Bindung oder Asp oder Glu darstellt,
A₃ Gly, Gln, Asn, Sar, Ala oder Ser bedeutet,
A₄ Phe oder N-Me-Phe darstellt,
A₅ Ile, Val, Leu, Phe, Ala, Tyr, Nle, Met oder N-Me-Val bedeutet;
A₆ Gly oder Sar bedeutet, und
Y einen Rest der Formel 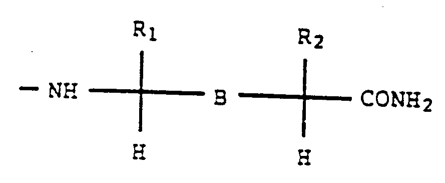 darstellt, wobei B einen Rest mit einer der folgenden Formeln darstellt 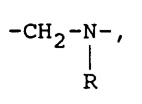 -CH₂-S-, -CH₂-O-, -CH=CH-, 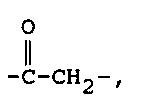 -CH(OH)CH₂-, oder 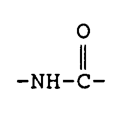 und wobei R ein Wasserstoffatom oder einen Alkylrest mit 1 bis 4 Kohlenstoffatomen oder einen Phenylalkylenrest darstellt, wobei die Alkyleneinheit geradkettig oder verzweigt ist und 1 bis 6 Kohlenstoffatome aufweist und wobei die Phenyleinheit unsubstituiert ist oder mit einem C₁₋₄-Alkylrest, C₁₋₄-Alkoxyrest, einer Hydroxylgruppe oder einem Halogenatom monosubstituiert ist,
R₁ und R₂ jeweils unabhängig voneinander ausgewählt sind aus Isopropyl-, Isobutyl-, sek.-Butyl-, n-Butyl- und 2-(Methylthio)ethylgruppen,
oder eines pharmazeutisch verträglichen Salzes davon, umfassend die Bindung an eine harzgebundene Verbindung der Formel 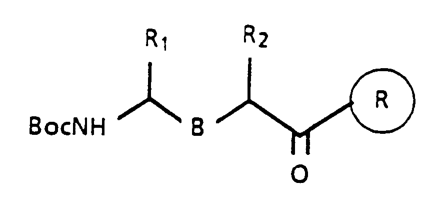 in der R₁, R₂ und B die in den vorangehenden Ansprüchen angegebene Bedeutung haben und wobei Boc eine Butyloxycarbonyl-Schutzgruppe ist und R (mit dem Kreis darum) das Harz darstellt, der α-aminogeschützten Aminosäuren in der Reihenfolge von A₆ bis zu A₁ an die terminale Aminogruppe der wachsenden Peptidkette, welche mittlerweile durch Entfernung ihrer Aminoschutzgruppe freigelegt wurde.

2. Verfahren nach Anspruch 1, wobei X ein Wasserstoffatom ist.

3. Verfahren nach Anspruch 1 oder 2, wobei A₁ eine Bindung und X Glt, Mal, Fum oder Suc bedeutet.

4. Verfahren nach einem der Ansprüche 1 bis 3, wobei A₁ eine Bindung und X Suc bedeutet.

5. Verfahren nach Anspruch 1, wobei A₁ His-Lys-Thr, Lys-Thr oder Thr bedeutet.

6. Verfahren nach einem der Ansprüche 1 bis 5, wobei A₂ Asp bedeutet.

7. Verfahren nach einem der Ansprüche 1 bis 6, wobei A₃ Ala oder Ser bedeutet.

8. Verfahren nach einem der Ansprüche 1 bis 7, wobei A₄ Phe bedeutet.

9. Verfahren nach einem der Ansprüche 1 bis 8, wobei A₅ Val bedeutet.

10. Verfahren nach einem der Ansprüche 1 bis 9, wobei A₆ Gly bedeutet.

11. Verfahren nach einem der Ansprüche 1 bis 10, wobei B 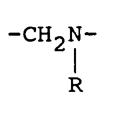 bedeutet.

12. Verfahren nach einem der Ansprüche 1 bis 11, wobei R₁ eine Isobutylgruppe ist.

13. Verfahren nach einem der Ansprüche 1 bis 12, wobei R₂ eine 2-Methylthioethyl-, Isobutyl- oder n-Butylgruppe bedeutet.

14. Verfahren nach einem der Ansprüche 1 bis 13, wobei R₁ und R₂ jeweils eine Isobutylgruppe bedeuten.

15. Verfahren nach Anspruch 1, wobei X ein Wasserstoffatom, Glt, Mal, Fum oder Suc bedeutet, A₁ eine Bindung, His-Lys-Thr, Lys-Thr, Thr, Val-Pro-Lys-Ser,Pro-Lys-Ser, Lys-Ser, Ser, pGlu-Pro-Ser-Lys, Pro-Ser-Lys, Ser-Lys oder Lys darstellt, A₂ eine Bindung, Asp oder Glu bedeutet, A₃ Gly, Gln, Asn, Sar, Ala oder Ser bedeutet, A₄ Phe oder N-Me-Phe bedeutet, A₅ Ile, Val, Leu, Phe, Ala, Tyr, Nle, Met oder N-Me-Val bedeutet, A₆ Gly oder Sar bedeutet, B -CH₂NH- bedeutet und R₁ und R₂ unabhängig voneinander ausgewählt sind aus einer Isopropyl-, Isobutyl-, sek.-Butyl-, n-Butyl- oder 2-(Methylthio)ethyl-Gruppe.

16. Verfahren nach Anspruch 15, wobei X ein Wasserstoffatom ist.

17. Verfahren nach Anspruch 15 oder 16, wobei A₁ eine Bindung, His-Lys-Thr, Lys-Thr oder Thr bedeutet.

18. Verfahren nach einem der Ansprüche 15 bis 17, wobei A₂ eine Bindung oder Asp bedeutet.

19. Verfahren nach einem der Ansprüche 15 bis 18, wobei X ein Wasserstoffatom darstellt, A₁ His-Lys-Thr, Lys-Thr oder Thr darstellt und A₂ Asp bedeutet.

20. Verfahren nach einem der Ansprüche 15 bis 19, wobei A₃ Ala oder Ser bedeutet.

21. Verfahren nach einem der Ansprüche 15 bis 20, wobei A₄ Phe bedeutet.

22. Verfahren nach einem der Ansprüche 15 bis 21, wobei A₅ Val bedeutet.

23. Verfahren nach einem der Ansprüche 15 bis 22, wobei A₆ Gly bedeutet.

24. Verfahren nach einem der Ansprüche 15 bis 23, wobei B 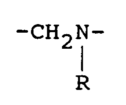 bedeutet.

25. Verfahren nach einem der Ansprüche 15 bis 24, wobei R₁ eine sek.-Butylgruppe ist.

26. Verfahren nach einem der Ansprüche 15 bis 25, wobei R₂ eine 2-(Methylthio)ethyl-, sek.-Butyl- oder n-Butylgruppe ist.

27. Verfahren nach Anspruch 15, wobei die erhaltene Verbindung
H-Asp-Ser-Phe-Val-Gly-LeuΨ[CH₂NH]Leu-NH₂ oder
H-Asp-Ser-Phe-Val-Gly-LeuΨ[CH₂N(CH₃)]Leu-NH₂
ist.

28. Verwendung eines Peptidderivats erhältlich gemäß einem Verfahren nach einem der Ansprüche 1 bis 27 für die Herstellung eines Arzneimittels.

29. Verwendung eines Peptidderivats erhältlich gemäß einem Verfahren nach einem der Ansprüche 1 bis 27 für die Behandlung von Asthma, Entzündungen und/oder Arthritis.

## Patentansprüche (Patentansprüche für folgende(n) Vertragsstaat(en): GR)

1. Peptidderivat der allgemeinen Formel I
X-A₁-A₂-A₃-A₄-A₅-A₆-Y (I)
in der X ein Wasserstoffatom, einen Alkylrest mit 1 bis 6 Kohlenstoffatomen oder einen Acylrest mit 2 bis 10 Kohlenstoffatomen bedeutet,
A₁ eine Bindung oder His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys oder His bedeutet,
A₂ eine Bindung oder Asp oder Glu darstellt,
A₃ Gly, Gln, Asn, Sar, Ala oder Ser bedeutet,
A₄ Phe oder N-Me-Phe darstellt,
A₅ Ile, Val, Leu, Phe, Ala, Tyr, Nle, Met oder N-Me-Val bedeutet;
A₆ Gly oder Sar bedeutet, und
Y einen Rest der Formel 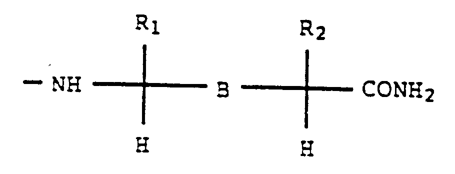 darstellt, wobei B einen Rest mit einer der folgenden Formeln darstellt 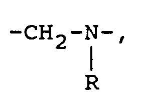 -CH₂-S-, -CH₂-O-, -CH=CH-, 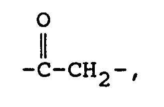 -CH(OH)CH₂-, oder 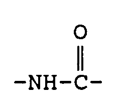 und wobei R ein Wasserstoffatom oder einen Alkylrest mit 1 bis 4 Kohlenstoffatomen oder einen Phenylalkylenrest darstellt, wobei die Alkyleneinheit geradkettig oder verzweigt ist und 1 bis 6 Kohlenstoffatome aufweist und wobei die Phenyleinheit unsubstituiert ist oder mit einem C₁₋₄-Alkylrest, C₁₋₄-Alkoxyrest, einer Hydroxylgruppe oder einem Halogenatom monosubstituiert ist,
R₁ und R₂ jeweils unabhängig voneinander ausgewählt sind aus Isopropyl-, Isobutyl-, sek.-Butyl-, n-Butyl- und 2-(Methylthio)ethylgruppen,
oder ein pharmazeutisch verträgliches Salz davon.

2. Peptidderivat nach Anspruch 1, wobei X ein Wasserstoffatom ist.

3. Peptidderivat nach Anspruch 1 oder 2, wobei A₁ eine Bindung und X Glt, Mal, Fum oder Suc bedeutet.

4. Peptidderivat nach einem der Ansprüche 1 bis 3, wobei A₁ eine Bindung und X Suc bedeutet.

5. Peptidderivat nach Anspruch 1, wobei A₁ His-Lys-Thr, Lys-Thr oder Thr bedeutet.

6. Peptidderivat nach einem der Ansprüche 1 bis 5, wobei A₂ Asp bedeutet.

7. Peptidderivat nach einem der Ansprüche 1 bis 6, wobei A₃ Ala oder Ser bedeutet.

8. Peptidderivat nach einem der Ansprüche 1 bis 7, wobei A₄ Phe bedeutet.

9. Peptidderivat nach einem der Ansprüche 1 bis 8, wobei A₅ Val bedeutet.

10. Peptidderivat nach einem der Ansprüche 1 bis 9, wobei A₆ Gly bedeutet.

11. Peptidderivat nach einem der Ansprüche 1 bis 10, wobei B 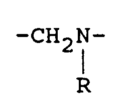 bedeutet.

12. Peptidderivat nach einem der Ansprüche 1 bis 11, wobei R₁ eine Isobutylgruppe ist.

13. Peptidderivat nach einem der Ansprüche 1 bis 12, wobei R₂ eine 2-Methylthioethyl-, Isobutyl- oder n-Butylgruppe bedeutet.

14. Peptidderivat nach einem der Ansprüche 1 bis 13, wobei R₁ und R₂ jeweils eine Isobutylgruppe bedeuten.

15. Peptidderivat nach Anspruch 1, wobei X ein Wasserstoffatom, Glt, Mal, Fum oder Suc bedeutet, A₁ eine Bindung, His-Lys-Thr, Lys-Thr, Thr, Val-Pro-Ly s-Ser,Pro-Lys-Ser, Lys-Ser, Ser, pGlu-Pro-Ser-Lys, Pro-Ser-Lys, Ser-Lys oder Lys darstellt, A₂ eine Bindung, Asp oder Glu bedeutet, A₃ Gly, Gln, Asn, Sar, Ala oder Ser bedeutet, A₄ Phe oder N-Me-Phe bedeutet, A₅ Ile, Val, Leu, Phe, Ala, Tyr, Nle, Met oder N-Me-Val bedeutet, A₆ Gly oder Sar bedeutet, B -CH₂NH- bedeutet und R₁ und R₂ unabhängig voneinander ausgewählt sind aus einer Isopropyl-, Isobutyl-, sek.-Butyl-, n-Butyl- oder 2-(Methylthio)ethyl-Gruppe.

16. Peptidderivat nach Anspruch 15, wobei X ein Wasserstoffatom ist.

17. Peptidderivat nach Anspruch 15 oder 16, wobei A₁ eine Bindung, His-Lys-Thr, Lys-Thr oder Thr bedeutet.

18. Peptidderivat nach einem der Ansprüche 15 bis 17, wobei A₂ eine Bindung oder Asp bedeutet.

19. Peptidderivat nach einem der Ansprüche 15 bis 18, wobei X ein Wasserstoffatom darstellt, A₁ His-Lys-Thr, Lys-Thr oder Thr darstellt und A₂ Asp bedeutet.

20. Peptidderivat nach einem der Ansprüche 15 bis 19, wobei A₃ Ala oder Ser bedeutet.

21. Peptidderivat nach einem der Ansprüche 15 bis 20, wobei A₄ Phe bedeutet.

22. Peptidderivat nach einem der Ansprüche 15 bis 21, wobei A₅ Val bedeutet.

23. Peptidderivat nach einem der Ansprüche 15 bis 22, wobei A₆ Gly bedeutet.

24. Peptidderivat nach einem der Ansprüche 15 bis 23, wobei B 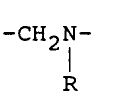 bedeutet.

25. Peptidderivat nach einem der Ansprüche 15 bis 24, wobei R₁ eine sek.-Butylgruppe ist.

26. Peptidderivat nach einem der Ansprüche 15 bis 25, wobei R₂ eine 2-(Methylthio)ethyl-, sek.-Butyl- oder n-Butylgruppe ist.

27. Peptidderivat nach Anspruch 15, nämlich
H-Asp-Ser-Phe-Val-Gly-LeuΨ[CH₂NH]Leu-NH₂ oder
H-Asp-Ser-Phe-Val-Gly-LeuΨ[CH₂N(CH₃)]Leu-NH₂.

28. Verfahren zur Herstellung eines Peptidderivats nach einem der Ansprüche 1 bis 27 oder eines pharmazeutisch verträglichen Salzes davon, umfassend die Bindung an eine harzgebundene Verbindung der Formel 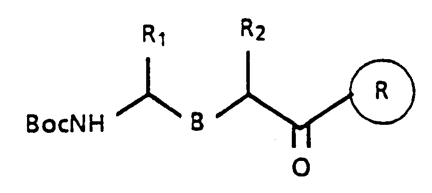 in der R₁, R₂ und B die in den vorangehenden Ansprüchen angegebene Bedeutung haben und wobei Boc eine Butyloxycarbonyl-Schutzgruppe ist und R (mit dem Kreis darum) das Harz darstellt, der α-aminogeschützten Aminosäuren in der Reihenfolge von A₆ bis zu A₁ an die terminale Aminogruppe der wachsenden Peptidkette, welche mittlerweile durch Entfernung ihrer Aminoschutzgruppe freigelegt wurde.

29. Peptidderivat nach einem der Ansprüche 1 bis 27 oder ein Gemisch davon zur Verwendung als pharmazeutisch wirksamer Stoff.

30. Peptidderivat nach einem der Ansprüche 1 bis 27 oder ein Gemisch davon zur Verwendung als Neurokinin A-Antagonist.

## Revendications (Revendications pour l'(les) Etat(s) contractant(s) suivant(s): AT, BE, CH, DE, FR, GB, IT, LI, LU, NL, SE)

1. Dérivé de peptide de formule générale I
X-A₁-A₂-A₃-A₄-A₅-A₆-Y (I)
dans laquelle
X est l'hydrogène, un groupe alkyle de 1 à 6 atomes de carbone ou un groupe acyle de 2 à 10 atomes de carbone;
A₁ est une liaison ou His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys ou His;
A₂ est une liaison ou est Asp ou Glu;
A₃ est Gly, Gln, Asn, Sar, Ala ou Ser;
A₄ est Phe ou N-Me-Phe;
A₅ est Ile, Val, Leu, Phe, Ala, Tyr, Nle, Met, ou N-Me-Val;
A₆ est Gly ou Sar; et
Y est un groupe de formule 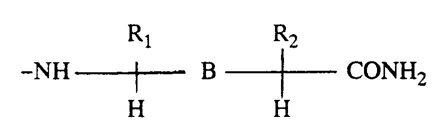 dans laquelle
B est un groupe ayant l'une des formules 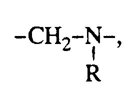 -CH₂-S-,
-CH₂-O-,
-CH=CH-, 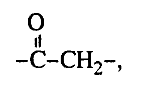 -CH(OH)CH₂-, ou 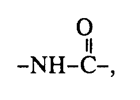 et où
R est un atome d'hydrogène ou un groupe alkyle de 1 à 4 atomes de carbone, ou est un groupe phénylalkylène dans lequel la partie alkylène est linéaire ou ramifiée et a de 1 à 6 atomes de carbone, et dans lequel la partie phényle est non substituée ou est monosubstituée par un groupe alkyle en C₁-C₄, alcoxy en C₁-C₄, hydroxy ou halogène;
R₁ et R₂ sont choisis chacun indépendamment parmi les groupes isopropyle, isobutyle, sec-butyle, n-butyle et 2-(méthylthio)éthyle,
ou un de ses sels pharmaceutiquement acceptables.

2. Dérivé de peptide selon la revendication 1, dans lequel X est l'hydrogène.

3. Dérivé de peptide selon la revendication 1 ou 2, dans lequel A₁ est une liaison et X est Glt, Mal, Fum ou Suc.

4. Dérivé de peptide selon l'une quelconque des revendications 1 à 3, dans lequel A₁ est une liaison et X est Suc.

5. Dérivé de peptide selon la revendication 1, dans lequel A₁ est His-Lys-Thr, Lys-Thr, ou Thr.

6. Dérivé de peptide selon l'une quelconque des revendications 1 à 5, dans lequel A₂ est Asp.

7. Dérivé de peptide selon l'une quelconque des revendications 1 à 6, dans lequel A₃ est Ala ou Ser.

8. Dérivé de peptide selon l'une quelconque des revendications 1 à 7, dans lequel A₄ est Phe.

9. Dérivé de peptide selon l'une quelconque des revendications 1 à 8, dans lequel A₅ est Val.

10. Dérivé de peptide selon l'une quelconque des revendications 1 à 9, dans lequel A₆ est Gly.

11. Dérivé de peptide selon l'une quelconque des revendications 1 à 10, dans lequel B est

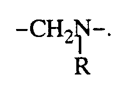

12. Dérivé de peptide selon l'une quelconque des revendications 1 à 11, dans lequel R₁ est isobutyle.

13. Dérivé de peptide selon l'une quelconque des revendications 1 à 12, dans lequel R₂ est 2-méthylthioéthyle, isobutyle ou n-butyle.

14. Dérivé de peptide selon l'une quelconque des revendications 1 à 13, dans lequel R₁ et R₂ sont chacun un isobutyle.

15. Dérivé de peptide selon la revendication 1, dans lequel X est l'hydrogène, Glt, Mal, Fum ou Suc; A₁ est une liaison, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys ou Lys; A₂ est une liaison, Asp ou Glu; A₃ est Gly, Gln, Asn, Sar, Ala ou Ser; A₄ est Phe ou N-Me-Phe; A₅ est Ile, Val, Leu, Phe, Ala, Tyr, Nle, Met ou N-Me-Val; A₆ est Gly ou Sar; B est -CH₂NH-; et R₁ et R₂ sont choisis chacun indépendamment parmi isopropyle, isobutyle, sec-butyle, n-butyle ou 2-(méthylthio)éthyle.

16. Dérivé de peptide selon la revendication 15, dans lequel X est l'hydrogène.

17. Dérivé de peptide selon la revendication 15 ou 16, dans lequel A₁ est une liaison, His-Lys-Thr, Lys-Thr ou Thr.

18. Dérivé de peptide selon l'une quelconque des revendications 15 à 17, dans lequel A₂ est une liaison ou Asp.

19. Dérivé de peptide selon l'une quelconque des revendications 15 à 18, dans lequel X est l'hydrogène; A₁ est His-Lys-Thr, Lys-Thr ou Thr; et A₂ est Asp.

20. Dérivé de peptide selon l'une quelconque des revendications 15 à 19, dans lequel A₃ est Ala ou Ser.

21. Dérivé de peptide selon l'une quelconque des revendications 15 à 20, dans lequel A₄ est Phe.

22. Dérivé de peptide selon l'une quelconque des revendications 15 à 21, dans lequel A₅ est Val.

23. Dérivé de peptide selon l'une quelconque des revendications 15 à 22, dans lequel A₆ est Gly.

24. Dérivé de peptide selon l'une quelconque des revendications 15 à 23, dans lequel B est

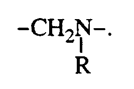

25. Dérivé de peptide selon l'une quelconque des revendications 15 à 24, dans lequel R₁ est sec-butyle.

26. Dérivé de peptide selon l'une quelconque des revendications 15 à 25, dans lequel R₂ est 2-(méthylthio)éthyle, sec-butyle ou n-butyle.

27. Dérivé de peptide selon la revendication 15, qui est
H-Asp-Ser-Phe-Val-Gly-LeuΨ[CH₂NH]Leu-NH₂ ou
H-Asp-Ser-Phe-Val-Gly-LeuΨ[CH₂N(CH₃)]Leu-NH₂.

28. Procédé de préparation d'un dérivé de peptide selon l'une quelconque des revendications 1 à 27 ou d'un de ses sels pharmaceutiquement acceptables, qui comprend la liaison, à un composé lié à une résine de formule 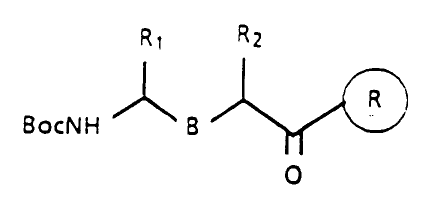 dans laquelle R₁, R₂ et B sont tels que définis dans les revendications précédentes et Boc est un groupe protecteur butyloxycarbonyle, et R (avec le cercle qui l'entoure) représente la résine, des aminoacides à groupe alpha amino protégé dans l'ordre de A₆ à A₁, au niveau du groupe amino terminal de la chaîne peptidique croissante qui a entre-temps été exposé par élimination de son groupe protecteur d'amino.

29. Dérivé de peptide selon l'une quelconque des revendications 1 à 27 ou un de ses mélanges pour l'utilisation comme substance pharmaceutiquement active.

30. Dérivé de peptide selon l'une quelconque des revendications 1 à 27 ou un de ses mélanges pour l'utilisation comme antagoniste de la neurokinine A.

31. Composition pharmaceutique pour le traitement de l'asthme, des inflammations et/ou de l'arthrite, contenant une quantité efficace d'un dérivé de peptide selon l'une quelconque des revendications 1 à 27 ou d'un de ses mélanges et éventuellement un support et/ou un diluant pharmaceutiquement acceptables.

## Revendications (Revendications pour l'(les) Etat(s) contractant(s) suivant(s): ES)

1. Procédé de préparation de dérivés de peptides de formule générale I
X-A₁-A₂-A₃-A₄-A₅-A₆-Y (I)
dans laquelle
X est l'hydrogène, un groupe alkyle de 1 à 6 atomes de carbone ou un groupe acyle de 2 à 10 atomes de carbone;
A₁ est une liaison ou His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys ou His;
A₂ est une liaison ou est Asp ou Glu;
A₃ est Gly, Gln, Asn, Sar, Ala ou Ser;
A₄ est Phe ou N-Me-Phe;
A₅ est Ile, Val, Leu, Phe, Ala, Tyr, Nle, Met, ou N-Me-Val;
A₆ est Gly ou Sar; et
Y est un groupe de formule 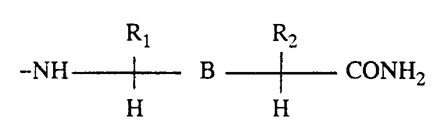 dans laquelle
B est un groupe ayant l'une des formules 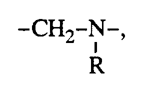 -CH₂-S-,
-CH₂-O-,
-CH=CH-, 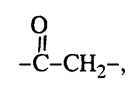 -CH(OH)CH₂-, ou 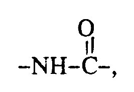 et où
R est un atome d'hydrogène ou un groupe alkyle de 1 à 4 atomes de carbone, ou est un groupe phénylalkylène dans lequel la partie alkylène est linéaire ou ramifiée et a de 1 à 6 atomes de carbone, et dans lequel la partie phényle est non substituée ou est monosubstituée par un groupe alkyle en C₁-C₄, alcoxy en C₁-C₄, hydroxy ou halogène;
R₁ et R₂ sont choisis chacun indépendamment parmi les groupes isopropyle, isobutyle, sec-butyle, n-butyle et 2-(méthylthio)éthyle,
ou un de ses sels pharmaceutiquement acceptables, qui comprend la liaison, à un composé lié à une résine de formule 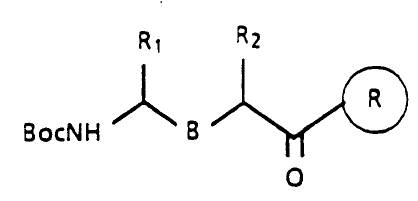 dans laquelle R₁, R₂ et B sont tels que définis ci-dessus et Boc est un groupe protecteur butyloxycarbonyle, et R (avec le cercle qui l'entoure) représente la résine, des aminoacides à groupe alpha amino protégé dans l'ordre de A₆ à A₁, au niveau du groupe amino terminal de la chaîne peptidique croissante qui a entre-temps été exposé par élimination de son groupe protecteur d'amino.

2. Procédé selon la revendication 1, dans lequel X est l'hydrogène.

3. Procédé selon la revendication 1 ou 2, dans lequel A₁ est une liaison et X est Glt, Mal, Fum ou Suc.

4. Procédé selon l'une quelconque des revendications 1 à 3, dans lequel A₁ est une liaison et X est Suc.

5. Procédé selon la revendication 1, dans lequel A₁ est His-Lys-Thr, Lys-Thr, ou Thr.

6. Procédé selon l'une quelconque des revendications 1 à 5, dans lequel A₂ est Asp.

7. Procédé selon l'une quelconque des revendications 1 à 6, dans lequel A₃ est Ala ou Ser.

8. Procédé selon l'une quelconque des revendications 1 à 7, dans lequel A₄ est Phe.

9. Procédé selon l'une quelconque des revendications 1 à 8, dans lequel A₅ est Val.

10. Procédé selon l'une quelconque des revendications 1 à 9, dans lequel A₆ est Gly.

11. Procédé selon l'une quelconque des revendications 1 à 10, dans lequel B est

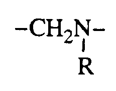

12. Procédé selon l'une quelconque des revendications 1 à 11, dans lequel R₁ est isobutyle.

13. Procédé selon l'une quelconque des revendications 1 à 12, dans lequel R₂ est 2-méthylthioéthyle, isobutyle ou n-butyle.

14. Procédé selon l'une quelconque des revendications 1 à 13, dans lequel R₁ et R₂ sont chacun un isobutyle.

15. Procédé selon la revendication 1, dans lequel X est l'hydrogène, Glt, Mal, Fum ou Suc; A₁ est une liaison, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys ou Lys; A₂ est une liaison, Asp ou Glu; A₃ est Gly, Gln, Asn, Sar, Ala ou Ser; A₄ est Phe ou N-Me-Phe; A₅ est Ile, Val, Leu, Phe, Ala, Tyr, Nle, Met ou N-Me-Val; A₆ est Gly ou Sar; B est -CH₂NH-; et R₁ et R₂ sont choisis chacun indépendamment parmi isopropyle, isobutyle, sec-butyle, n-butyle ou 2-(méthylthio)éthyle.

16. Procédé selon la revendication 15, dans lequel X est l'hydrogène.

17. Procédé selon la revendication 15 ou 16, dans lequel A₁ est une liaison, His-Lys-Thr, Lys-Thr ou Thr.

18. Procédé selon l'une quelconque des revendications 15 à 17, dans lequel A₂ est une liaison ou Asp.

19. Procédé selon l'une quelconque des revendications 15 à 18, dans lequel X est l'hydrogène; A₁ est His-Lys-Thr, Lys-Thr ou Thr; et A₂ est Asp.

20. Procédé selon l'une quelconque des revendications 15 à 19, dans lequel A₃ est Ala ou Ser.

21. Procédé selon l'une quelconque des revendications 15 à 20, dans lequel A₄ est Phe.

22. Procédé selon l'une quelconque des revendications 15 à 21, dans lequel A₅ est Val.

23. Procédé selon l'une quelconque des revendications 15 à 22, dans lequel A₆ est Gly.

24. Procédé selon l'une quelconque des revendications 15 à 23, dans lequel B est

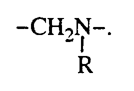

25. Procédé selon l'une quelconque des revendications 15 à 24, dans lequel R₁ est sec-butyle.

26. Procédé selon l'une quelconque des revendications 15 à 25, dans lequel R₂ est 2-(méthylthio)éthyle, sec-butyle ou n-butyle.

27. Procédé selon la revendication 15, dans lequel le composé obtenu est
H-Asp-Ser-Phe-Val-Gly-LeuΨ[CH₂NH]Leu-NH₂ ou
H-Asp-Ser-Phe-Val-Gly-LeuΨ[CH₂N(CH₃)]Leu-NH₂.

28. Utilisation d'un dérivé de peptide pouvant être obtenu par un procédé selon l'une quelconque des revendications 1 à 27, pour la préparation d'une composition pharmaceutique.

29. Utilisation d'un dérivé de peptide pouvant être obtenu par un procédé selon l'une quelconque des revendications 1 à 27, pour la préparation d'une composition pharmaceutique pour le traitement de l'asthme, des inflammations et de l'arthrite.

## Revendications (Revendications pour l'(les) Etat(s) contractant(s) suivant(s): GR)

1. Dérivé de peptide de formule générale I
X-A₁-A₂-A₃-A₄-A₅-A₆-Y (I)
dans laquelle
X est l'hydrogène, un groupe alkyle de 1 à 6 atomes de carbone ou un groupe acyle de 2 à 10 atomes de carbone;
A₁ est une liaison ou His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys, Lys ou His;
A₂ est une liaison ou est Asp ou Glu;
A₃ est Gly, Gln, Asn, Sar, Ala ou Ser;
A₄ est Phe ou N-Me-Phe;
A₅ est Ile, Val, Leu, Phe, Ala, Tyr, Nle, Met, ou N-Me-Val;
A₆ est Gly ou Sar; et
Y est un groupe de formule 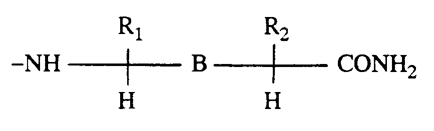 dans laquelle
B est un groupe ayant l'une des formules 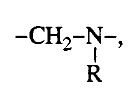 -CH₂-S-,
-CH₂-O-,
-CH=CH-, 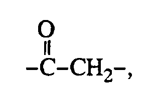 -CH(OH)CH₂-, ou 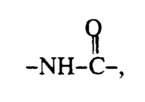 et où
R est un atome d'hydrogène ou un groupe alkyle de 1 à 4 atomes de carbone, ou est un groupe phénylalkylène dans lequel la partie alkylène est linéaire ou ramifiée et a de 1 à 6 atomes de carbone, et dans lequel la partie phényle est non substituée ou est monosubstituée par un groupe alkyle en C₁-C₄, alcoxy en C₁-C₄, hydroxy ou halogène;
R₁ et R₂ sont choisis chacun indépendamment parmi les groupes isopropyle, isobutyle, sec-butyle, n-butyle et 2-(méthylthio)éthyle,
ou un de ses sels pharmaceutiquement acceptables.

2. Dérivé de peptide selon la revendication 1, dans lequel X est l'hydrogène.

3. Dérivé de peptide selon la revendication 1 ou 2, dans lequel A₁ est une liaison et X est Glt, Mal, Fum ou Suc.

4. Dérivé de peptide selon l'une quelconque des revendications 1 à 3, dans lequel A₁ est une liaison et X est Suc.

5. Dérivé de peptide selon la revendication 1, dans lequel A₁ est His-Lys-Thr, Lys-Thr, ou Thr.

6. Dérivé de peptide selon l'une quelconque des revendications 1 à 5, dans lequel A₂ est Asp.

7. Dérivé de peptide selon l'une quelconque des revendications 1 à 6, dans lequel A₃ est Ala ou Ser.

8. Dérivé de peptide selon l'une quelconque des revendications 1 à 7, dans lequel A₄ est Phe.

9. Dérivé de peptide selon l'une quelconque des revendications 1 à 8, dans lequel A₅ est Val.

10. Dérivé de peptide selon l'une quelconque des revendications 1 à 9, dans lequel A₆ est Gly.

11. Dérivé de peptide selon l'une quelconque des revendications 1 à 10, dans lequel B est

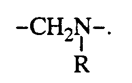

12. Dérivé de peptide selon l'une quelconque des revendications 1 à 11, dans lequel R₁ est isobutyle.

13. Dérivé de peptide selon l'une quelconque des revendications 1 à 12, dans lequel R₂ est 2-méthylthioéthyle, isobutyle ou n-butyle.

14. Dérivé de peptide selon l'une quelconque des revendications 1 à 13, dans lequel R₁ et R₂ sont chacun un isobutyle.

15. Dérivé de peptide selon la revendication 1, dans lequel X est l'hydrogène, Glt, Mal, Fum ou Suc; A₁ est une liaison, His-Lys-Thr, Lys-Thr, Thr, Val-Pro-Lys-Ser, Pro-Lys-Ser, Lys-Ser, Ser, pGlu-Pro-Ser-Lys, Pro-Ser-Lys, Ser-Lys ou Lys; A₂ est une liaison, Asp ou Glu; A₃ est Gly, Gln, Asn, Sar, Ala ou Ser; A₄ est Phe ou N-Me-Phe; A₅ est Ile, Val, Leu, Phe, Ala, Tyr, Nle, Met ou N-Me-Val; A₆ est Gly ou Sar; B est -CH₂NH-; et R₁ et R₂ sont choisis chacun indépendamment parmi isopropyle, isobutyle, sec-butyle, n-butyle ou 2-(méthylthio)éthyle.

16. Dérivé de peptide selon la revendication 15, dans lequel X est l'hydrogène.

17. Dérivé de peptide selon la revendication 15 ou 16, dans lequel A₁ est une liaison, His-Lys-Thr, Lys-Thr ou Thr.

18. Dérivé de peptide selon l'une quelconque des revendications 15 à 17, dans lequel A₂ est une liaison ou Asp.

19. Dérivé de peptide selon l'une quelconque des revendications 15 à 18, dans lequel X est l'hydrogène; A₁ est His-Lys-Thr, Lys-Thr ou Thr; et A₂ est Asp.

20. Dérivé de peptide selon l'une quelconque des revendications 15 à 19, dans lequel A₃ est Ala ou Ser.

21. Dérivé de peptide selon l'une quelconque des revendications 15 à 20, dans lequel A₄ est Phe.

22. Dérivé de peptide selon l'une quelconque des revendications 15 à 21, dans lequel A₅ est Val.

23. Dérivé de peptide selon l'une quelconque des revendications 15 à 22, dans lequel A₆ est Gly.

24. Dérivé de peptide selon l'une quelconque des revendications 15 à 23, dans lequel B est

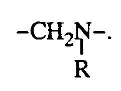

25. Dérivé de peptide selon l'une quelconque des revendications 15 à 24, dans lequel R₁ est sec-butyle.

26. Dérivé de peptide selon l'une quelconque des revendications 15 à 25, dans lequel R₂ est 2-(méthylthio)éthyle, sec-butyle ou n-butyle.

27. Dérivé de peptide selon la revendication 15, qui est
H-Asp-Ser-Phe-Val-Gly-LeuΨ[CH₂NH]Leu-NH₂ ou
H-Asp-Ser-Phe-Val-Gly-LeuΨ[CH₂N(CH₃)]Leu-NH₂.

28. Procédé de préparation d'un dérivé de peptide selon l'une quelconque des revendications 1 à 27 ou d'un de ses sels pharmaceutiquement acceptables, qui comprend la liaison, à un composé lié à une résine de formule 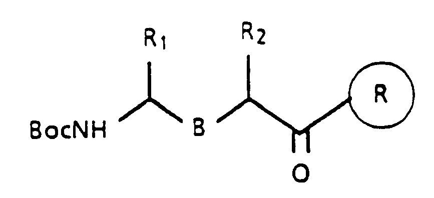 dans laquelle R₁, R₂ et B sont tels que définis dans les revendications précédentes et Boc est un groupe protecteur butyloxycarbonyle, et R (avec le cercle qui l'entoure) représente la résine, des aminoacides à groupe alpha amino protégé dans l'ordre de A₆ à A₁, au niveau du groupe amino terminal de la chaîne peptidique croissante qui a entre-temps été exposé par élimination de son groupe protecteur d'amino.

29. Dérivé de peptide selon l'une quelconque des revendications 1 à 27 ou un de ses mélanges pour l'utilisation comme substance pharmaceutiquement active.

30. Dérivé de peptide selon l'une quelconque des revendications 1 à 27 ou un de ses mélanges pour l'utilisation comme antagoniste de la neurokinine A.
